(19) Europäisches Patentamt
European Patent Office
Office européen des brevets

(11) EP 1 098 257 A2

(12) EUROPEAN PATENT APPLICATION

(43) Date of publication:
09.05.2001 Bulletin 2001/19

(51) Int Cl.$^7$: G06F 17/30, G06F 19/00

(21) Application number: 00309830.8

(22) Date of filing: 06.11.2000

(84) Designated Contracting States:
AT BE CH CY DE DK ES FI FR GB GR IE IT LI LU
MC NL PT SE TR
Designated Extension States:
AL LT LV MK RO SI

(30) Priority: 08.11.1999 JP 31734299

(71) Applicant: Biomolecular Engineering Research
Institute
Suita-shi, Osaka 565-0874 (JP)

(72) Inventors:
• Hiroike, Takaaki
  Higashiosaka-shi, osaka 577-0813 (JP)
• Toh, Hiroyuki
  Suita-shi, Osaka 565-0882 (JP)

(74) Representative:
Howden, Christopher Andrew et al
FORRESTER & BOEHMERT
Franz-Joseph-Strasse 38
80801 München (DE)

(54) **Structural local alignment method using a double dynamic programming algorithm**

(57) The present invention discloses a structural local alignment method for proteins using a double dynamic programming algorithm enabling carrying out proper alignment of not only those proteins having a partially similar structure, which proteins are not similar to one another in terms of overall structure, but also those proteins in circular permutation one another. The alignment method of the invention is devised to maintain constant presentation of the structural environments even when an exchange between the N-terminal and C-terminal regions takes place. To this end, upon determination of a structural environment at amino acid residue i, N-terminal and C-terminal vector sets are established independently, the N-terminal vectors being apposed in order of amino acid residue No., and the C-terminal vectors being apposed in order of amino acid residue No.

F I G. 4

PROTEIN A

PROTEIN B

PROTEIN B

PROTEIN A

SECOND HIGHEST
SCORE

THIRD HIGHEST
SCORE

THE HIGHEST SCORE

## Description

## BACKGROUND OF THE INVENTION

The present invention relates to a structural local alignment method for proteins using a double dynamic programming algorithm for comparative analysis of the tertiary structures of proteins, with an aim toward obtaining information about protein structures and functions as well as molecular evolution. Concerning the related references in this field, the following references have been published:

(1) C.A. Orengo & W.R. Taylor: J. Mol. Biol. (1993), 233, 488-497.
(2) T.F. Smith & M.S. Waterman: J. Mol. Biol. (1981), 147, 195-197.
(3) Toh, H.: CABIOS (1997), 13, 387-396.

In concert with advances in techniques used for determining the primary and tertiary structures of proteins, the databases for amino acid sequences and tertiary structures have grown rapidly. Knowledge of life is being obtained by handling such a vast amount of information; so-called bioinformatics technology.

In particular, in the field of medical treatment, there are moves to facilitate more effective development of treatment-related medical materials by using this vast amount of information. In terms of experimental technique, it is relatively quick and easy to determine the sequences of proteins. In contrast, it requires a great deal of time and manpower to obtain information as to whether the protein could be a target worth developing for treatment-related medical materials; that is, it is difficult to obtain molecular information relating to in vivo functions that are required for producing drugs as well as information relating to the agonists and antagonists of the protein. Therefore, when a protein which has an unknown function and is specifically expressed at a certain disease site is discovered, an attempt is made to predict the function of the protein by comparing, using databases, the sequence of the unknown protein with the sequences of other proteins of known functions, and deducing the function of the protein from that of homologous proteins having similar sequences. By predicting the function of the protein, we can select the experiments to confirm the function and screen the antagonists and the agonists for the protein. Further, the disease mechanism can be hypothesized from the protein function, thus leading to determination of whether or not the protein is suitable for developing treatment-related medical materials or worth developing as a diagnostic drug.

In the case of a protein with an unknown tertiary structure, if its homologous protein with a known tertiary structure is found from sequence comparison, the tertiary structure of the target protein can be constructed using the known tertiary structure as a template. Further, rational drug designs can be formulated from the thus-constructed structure. Recently, however, some proteins that have a low degree of similarity in the amino acid sequence with one another have been found to have functions similar to one another and have tertiary structures similar to one another. Thus, it has become necessary to examine not only the comparisons at the sequence level, i.e. to understand relationships between the sequences and the functions, but also the comparisons at the tertiary structure level, i.e. relationships between the tertiary structures and the functions.

In comparative analysis, there is an important basic technique called alignment. Usually, an alignment between amino acid sequences or nucleotide sequences is called a sequence alignment, whereas an alignment on the tertiary structure level is called a structural alignment. To carry out alignment means that, when there are two homologous sequences or structures, the residues in the sequences or structures are apposed according to their corresponding equivalence. In addition, if there is no corresponding residue in one sequence or one structure, an operation is carried out in such a way that an emptiness-indicating symbol called a gap is introduced into the corresponding empty position.

There are also two types of alignment methods: the first is called global alignment wherein the entire protein region is compared, and the other is local alignment for apposing only the region that is selected for homology. If the proteins have a high overall similarity to one another, then their comparative analysis can be carried out by the global alignment method. However, if they do not have an overall similarity but only have partial similarity in their sequences or structures, then the local alignment method will have to be used. The significance of finding local similarity of proteins is as follows:

(1) If proteins have the same function, even when they have low overall homology, the regions related to the function have comparatively high homology in many cases.
(2) In the case of a large protein, the protein is often constituted by multiple functioning structures (functional domains), each of which has one function.
(3) When circular permutation occurs to one functional domain in a protein, the circular permuted protein contains the functional domain in the reverse order between the NH2-terminal sequence and the COOH-terminal sequence against the original one. In order to analyze such circular permuted proteins, comparative analyses of local structures, rather than analyses of overall structures, are appropriate, since these proteins have the similarities between the N-terminal region of one protein and the C-terminal region of the other protein, and between the C-terminal region of one protein and the N-terminal region of the other protein.

The structural local alignment related to the present invention is a new alignment solution sought in response to the above-described necessity of carrying out comparative analyses at the tertiary structure level. The basic methodology was developed by C.A. Orengo and W.R. Taylor in 1993 using a double dynamic programming algorithm (J. Mol. Biol. 1993, 233, 488-497). The double dynamic programming is one of algorithms capable of handling tertiary structures developed by expanding the dynamic programming algorithm, which is a representative algorithm to obtain the best-matched sequence alignment between two proteins.

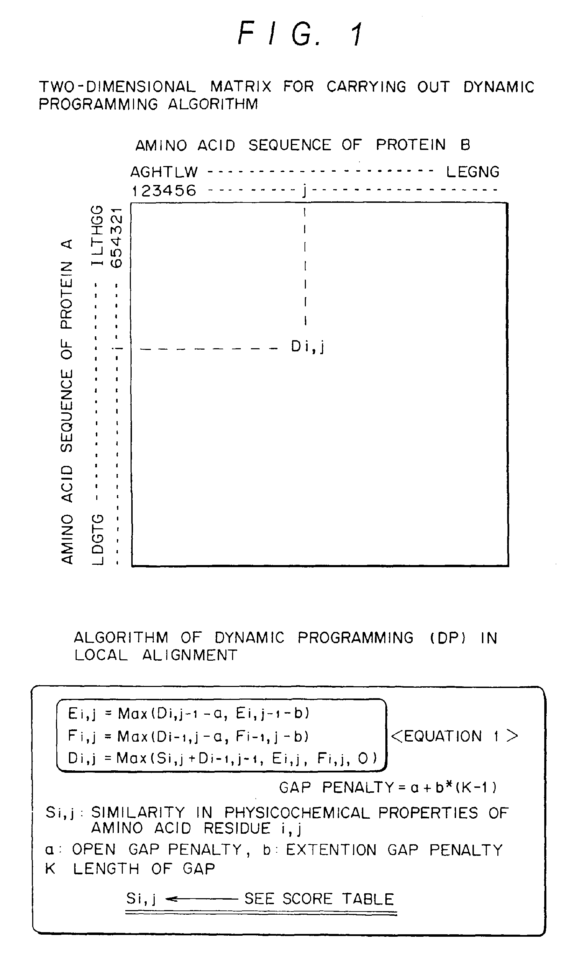

To elucidate the double dynamic programming algorithm, we will first refer to its prototype, the dynamic programming algorithm (T.F. Smith and M.S. Waterman: J. Mol. Biol. (1981), 147, 195-197) used for local sequence alignment. As shown in FIG. 1, to carry out the dynamic programming algorithm, a 2 dimensional matrix is prepared. The amino acid sequence of protein A on the matrix column side and that of protein B on the row are arranged, respectively. Using equation (1) shown below, from the terminal residue pair at either end of proteins A and B toward the other end, the value at each element; i.e., ($D_{i,j}$), on the matrix is determined one by one in sequence.

$$E_{i,j} = Max\ (D_{i,j-1} - a,\ E_{i,j-1} - b)$$

$$F_{i,j} = Max\ (D_{i-1,j} - a,\ F_{i-1,j} - b)$$

$$D_{i,j} = Max\ (S_{i,j} + D_{i-1,j-1},\ E_{i,j},\ F_{i,j},\ 0) \qquad (1)$$

where $S_{i,j}$ is a physicochemical similarity score between amino acid residues i and j, a is an open gap penalty, and b is an extension gap penalty.

The significance of the above equation (1) is to adopt $D_{i,j}$ as the maximum value among the four arguments, $S_{i,j} + D_{i-1,j-1}$, $E_{i,j}$, $F_{i,j}$, and 0. Here, a and b are arbitrary constants called gap penalties, of which the former is a penalty value (an open gap penalty) to allow introduction of the first gap and the latter is a penalty value (an extension gap penalty) to further allow introduction of an extended gap. $S_{i,j}$ is either a negative or positive value, and its value can be obtained from a table, called a score table, indicating the similarities of every amino acid pair.

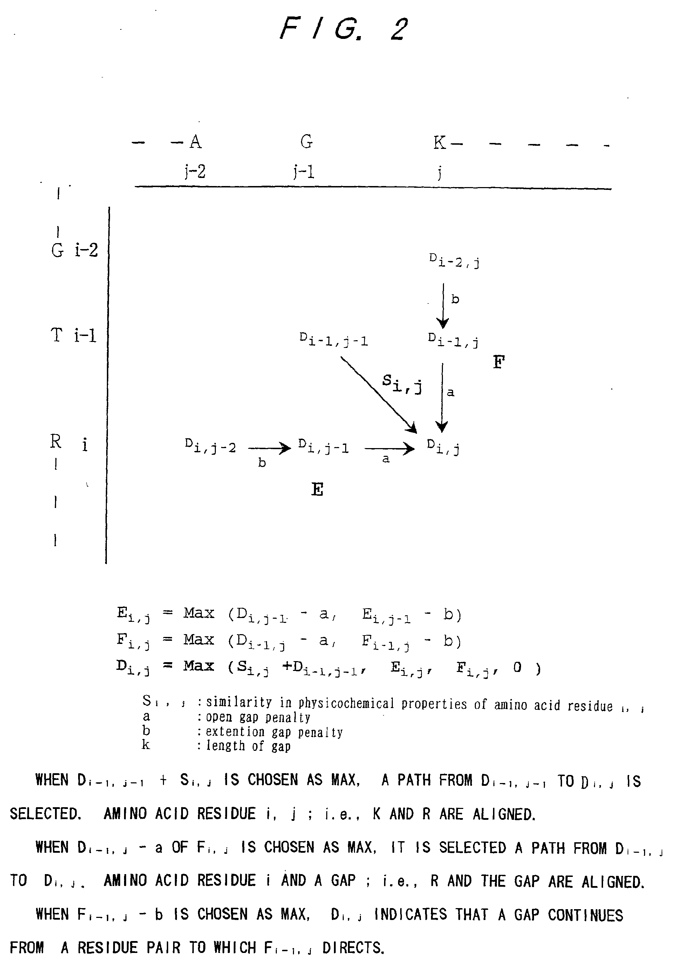

The representative score table is Dayhoff's score table (FIG. 2), a numerical table, giving a large positive value to a pair of amino acids with high physicochemical similarities and a small negative value to those not similar to each other. In other words, $S_{i,j}$ shows a similarity of a residue pair between two protein by using the score table values.

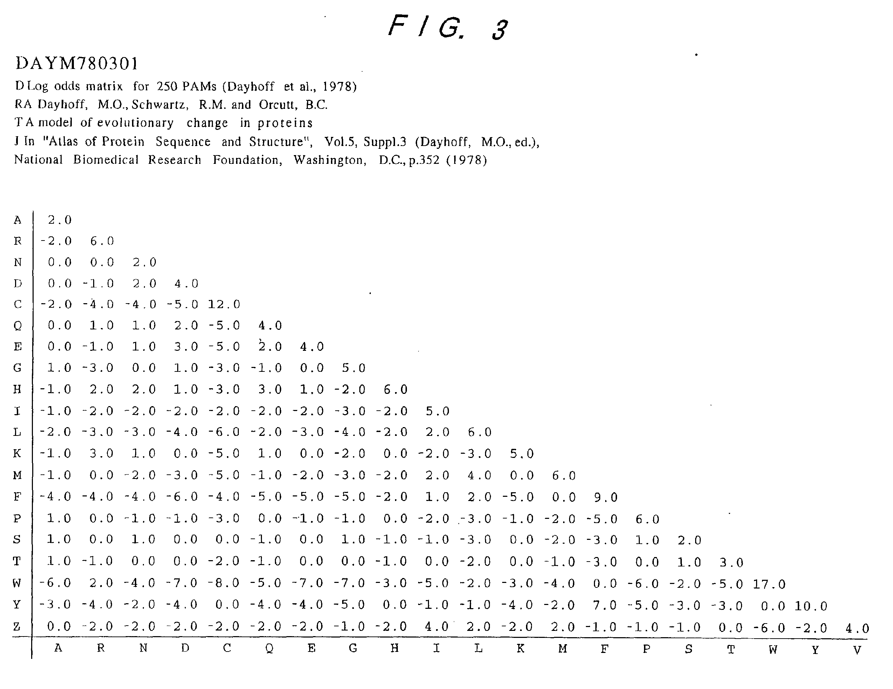

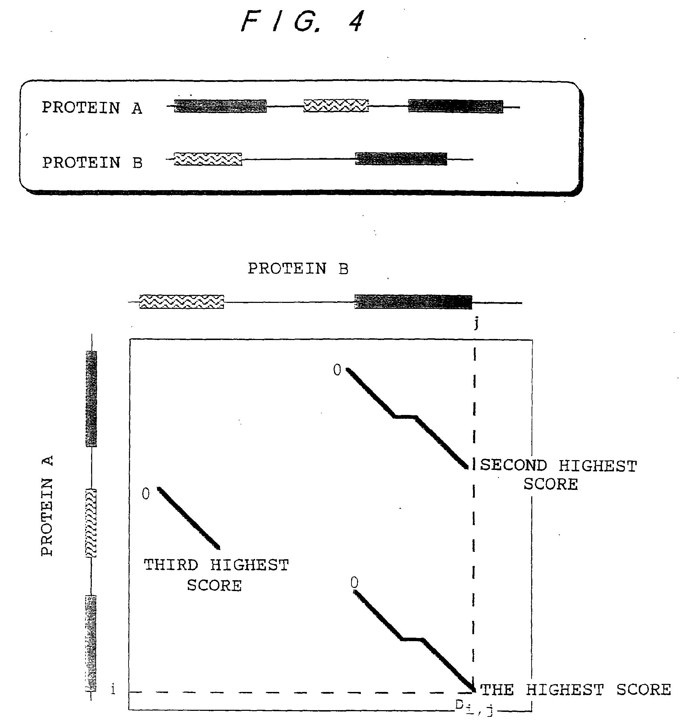

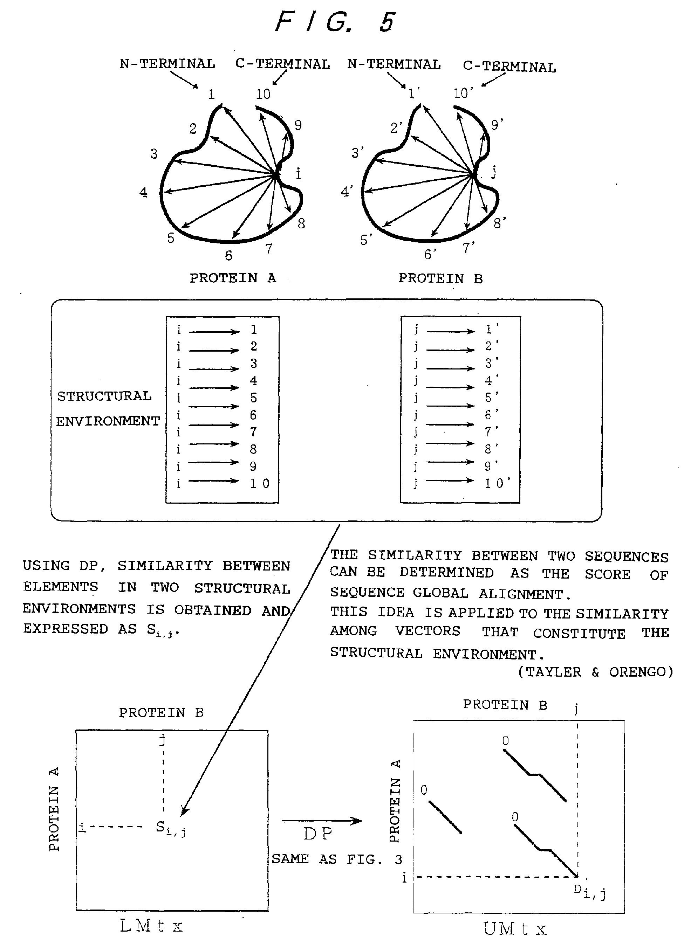

When the maximum value $D_{i,j}$ is $S_{i,j} + D_{i-1,j-1}$, this indicates that an alignment with the pairing amino acid residues i and j gives the highest score, thus generating a diagonal path from $D_{i-1,j-1}$ to $D_{i,j}$ within the matrix in FIG. 3 (corresponding to an arrow $\rightarrow$ in the schematic diagram for the recurrence equation (FIG. 3)). Adoption of $E_{i,j}$ or $F_{i,j}$ indicates an alignment by pairing residue i and a gap or by pairing residue j and a gap, thus generating a parallel or vertical path, respectively.

Further, $E_{i,j} = Max\ (D_{i,j-1} - a,\ E_{i,j-1} - b)$ or $F_{i,j} = Max\ (D_{i-1,j} - a,\ F_{i-1,j} - b)$ respectively determines whether the open or extension gap penalty should be adopted as a penalty.

In the case of the open gap penalty, a path from $D_{i,j-1}$ or $D_{i-1,j}$ to $D_{i,j}$ is generated as shown in FIG. 3, whereas in the case of the extension gap penalty the path is extended to $D_{i,j}$ from the path-starting base pair indicated by $E_{i,j-1}$ or $F_{i-1,j}$.

Moreover, if $D_{i,j}$ becomes a negative value, zero is inserted into $D_{i,j}$ by the Max zero term operation so that the path is not extended any further. Thus, due to the Max zero term operation, a new alignment can start from an arbitrary point where $D_{i,j}$ is zero, it thereby becoming possible to generate two or more local alignments.

Upon computation of the above equation (1), the path selected by the Max operation is registered together with the score ($D_{i,j}$). Next, after finishing computation of all the residues using the recurrence equation (1), an operation called backtracking is performed. In other words, from the pairing of residues i andj showing the maximum scoring value, backtracking is carried out following the path where the maximum value has been identified by the above equation (1), thus obtaining the local alignment. The backtracking will end at a position where the Max becomes zero.

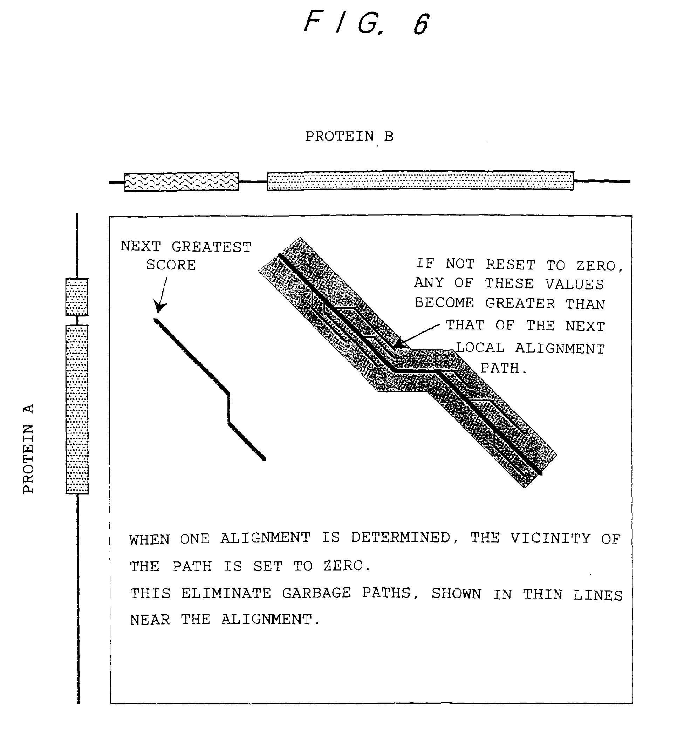

Further, from a position of a residue pair giving the next highest score value, backtracking can be carried out again, it thus becoming possible to obtain the second local alignment (see FIG. 4). Also, after the second alignment, further alignments can be obtained similarly. Another advanced algorithm using this strategy is the dynamic programming algorithm for local sequence alignment developed by Smith and Waterman.

The basic methodology for structural local alignment relating to the present invention uses essentially the same dynamic programming algorithm that was used in Smith and Waterman's local alignment methodology as described above. In other words, upon performing the dynamic programming algorithm, if a structural similarity value is given to each residue pair between the two proteins, instead of the scoring table (see FIG. 2) indicating the amino acid similarity scores for the $S_{i,j}$ values as shown in FIGs. 1 and 3, the structural local alignment can be obtained with ease by using the same algorithm shown in the above equation (1). For this, Taylor and Orengo proposed a structural environment as a value for structural similarity (see FIG. 5).

The structural environment of residue i in protein A is expressed as a set of vectors aligned in order of residue number after obtaining each vector (or distance) from a particular atom (Cβ) in the residue i to particular atoms (Cβ) in all the other amino acid residues. In other words, the structural environment of residue i shows the relative position of residue i for all the other amino acid residues in protein A. Thus, the structural similarity between residue i in protein A and residue j in protein B can be determined by a structural environment similarity between residues i and j.

Here, since the structural environment is a set of one dimensional vector data, the structural similarity between residues i and j can be handled in the same manner as the determination of sequence similarity. Therefore, Taylor and Orengo proposed that the global alignment score between the structural environment of residue i and that of residue j represents a structural environment similarity $S_{i,j}$ at residues i and j, the score being obtained by using the dynamic programming algorithm (see FIG. 5). Thus, the structural local alignment can be obtained by solving the above equation (1) using the above obtained $S_{i,j}$ value.

As described above, the dynamic programming algorithm is used twice, first for determination of the structural environment similarity (lower level DP) and again for solving the structural local alignment (upper level DP). Thus, the methodology for structural local alignment is called the double dynamic programming algorithm.

FIG. 6 depicts a schematic view of the structural local alignment. Surrounding an aligned path, there are many paths; and, by following those paths, many of them turn out to merge with the original aligned path. Because of this, as shown in FIG. 4, for obtaining local alignments subsequent to the second local alignment, there is an operation excluding $D_{i,j}$ that merges into the first alignment by exchanging the score of each residue pair surrounding the already aligned path by upper level DP to zero. Thus, the structural local alignment is obtained by the double dynamic programming algorithm developed by Taylor and Orengo.

Using the double dynamic programming algorithm, determination of the local alignment became possible not just at the sequence level of a protein but at the tertiary structure level. However, the problem remains that accurate alignments still cannot be obtained if the proteins are in the circular permutation relationship with one another.

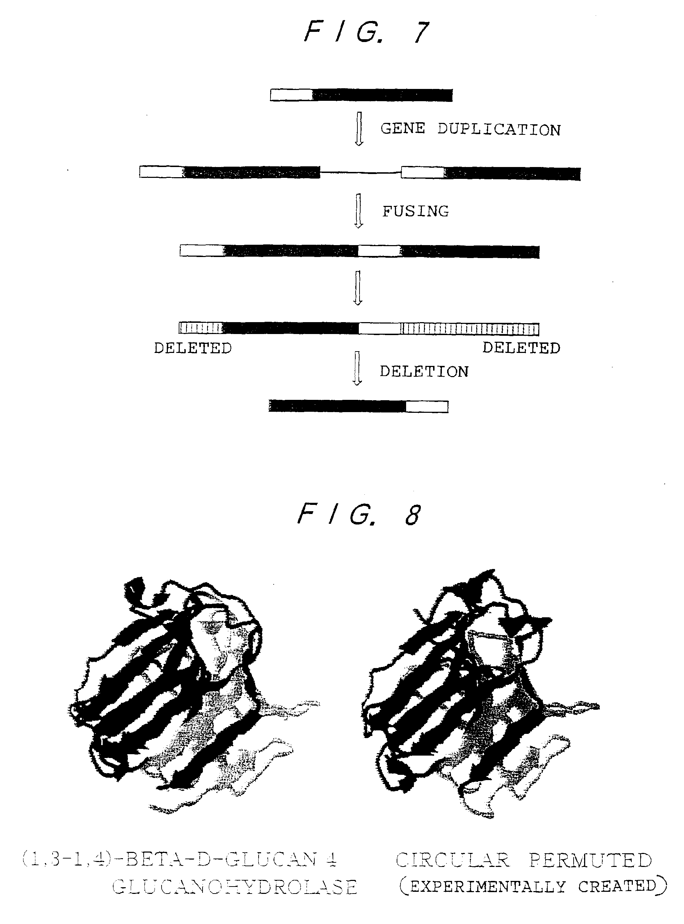

As shown in FIG. 7, circular permutation occurs in the following manner: first two identical genes that have the same sequences are generated by gene duplication, fuse together; and then, due to deletions at both ends, a new gene is generated, encoding a protein whose N-terminal and C-terminal regions in the original protein have been interchanged. In particular, if the residue of the N-terminal is close in spatial terms to that of the C-terminal in a protein, the same function could be maintained without overall difference between the original structure and the structure resulting from circular permutation.

FIG. 8 shows an example of circular permutation for glucanase, indicating that the overall tertiary structure is almost identical between the non-permuted and permuted glucanases. In the above case, circular permuted proteins which were generated artificially are shown. However, there are examples of circular permutation occurring in nature, such as human glutathione synthetase and bacterial glutathione synthetase (The EMBO Journal 1999 18 (12): 3204-3213), aldolase and transaldolase (Structure 1996 Jun 15; 4 (6): 715-724), and FMN-binding protein and the FAD binding domain of phthalate dioxygenase reductase (Nat Struct. Biol. 1998 Feb; 5 (2): 101).

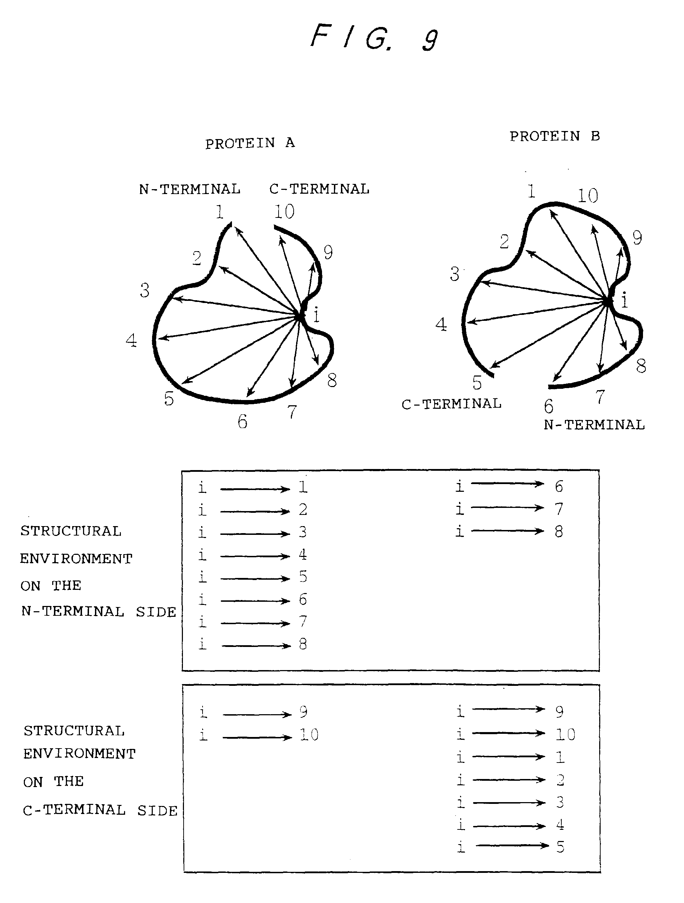

The structural local alignment method developed by Orengo and Taylor has the problem as follows. In the case of proteins related to circular permutation, when their corresponding amino acid residues in structurally identical regions are expressed using structural environments, the structural environments of the proteins are expressed as different vector sets as shown in FIG. 9, and thus the tertiary structural similarity between the proteins is considered to be low. Upon construction of the structural environment, the component vectors are aligned in order of residue No. from the N-terminus to C-terminus, thereby leading to construction of a structural environment depending on the order of residue No. Because of this dependency, structural environments at a structurally similar region in the proteins, whose N-terminal and C-terminal amino acid sequences are interchanged, cannot be accurately evaluated.

FIG. 9 is described in more detail. Provided that two proteins A and B have a circular permutation relationship to each other, then residues shown with the same numbers are corresponding residues at similar structural regions of the two proteins. Using the Taylor and Orengo method, components of a structural environment at residue i in protein A are aligned in order of 1, 2, 3, 4, 5, 6, 7, 8, 9, and 10, whereas those at residue i in protein B are in order of 6, 7, 8, 9, 10, 1, 2, 3, 4, and 5.

Where the proteins have a circular permutation relationship, the N-terminal region of one protein and the C-terminal region of the other protein (or vice versa) are expected to be locally aligned as similar structures. This kind of problem can be handled only by local alignment methods. However, the problem remains that the structural local alignment method cannot handle proteins in a circular permutation relationship, as mentioned above.

Considering the above problem, we have achieved the present invention with an aim toward providing a structural local alignment method capable of correctly aligning even those proteins having a circular permutation relationship through use of the double dynamic programming algorithm.

## SUMMARY OF THE INVENTION

Accordingly, the present invention provides, for maintaining constant presentation of the structural environments even when an exchange between the N-terminal and C-terminal regions takes place, a structural local alignment method using the double dynamic programming algorithm, which method comprising:

(a) determining a structural environment as a vector directed from a particular atom (Cα or Cβ) in each amino acid residue to a particular atom (Cα or Cβ) in all the other amino acid residues;

(b) wherein in the above mentioned step (a), upon determination of a structural environment at amino acid residue i, establishing N-terminal and C-terminal vector sets, the N-terminal vectors being apposed in order of amino acid residue No. from a vector for the N-terminal residue to that for amino acid residue i - 1, and the C-terminal vectors being apposed in order of amino acid residue No. from a vector for amino acid residue i + 1 to that for the C-terminal;

(c) reconstructing the structural environment so that the C-terminal vector set comes first and the N-terminal vector set comes next;

(d) obtaining a first structural local alignment with the double dynamic programming algorithm using the structural environment constructed in the above step (c) ;

(e) resetting, after obtaining the above first structural local alignment, the similarity value of structural environment ($S_{i,j}$) to an anomalous value for each residue within a 5-10 residue width before and after a residue pair on the path;

(f) determining the second structural local alignment by backtracking after re-computing the upper level DP using the structural environment similarity values reset at the above step (e) while omitting pairing residues with $S_{i,j}$ that has been reset to the anomalous value; and

(g) obtaining further structural local alignments by repeating the operation of the above steps (e) and (f) until the maximum score falls below the set value.

(2) The present invention also provides the above-described structural local alignment method using the double dynamic programming algorithm, which employs a distance cut-off operation for reducing computing time, the operation taking into consideration the protein local structure in the form of a structural environment; and secondary structure information of protein for improving alignment accuracy.

Thus, the present invention provides a method which generates structural local alignments even for the proteins involving circular permutation.

## BRIEF DESCRIPTION OF THE DRAWINGS

FIG. 1 shows the two dimensional matrix and the algorithm for dynamic programming used to carry out the dynamic programming algorithm.

FIG. 2 shows an example of a score table.

FIG. 3 shows the two dimensional matrix used to perform the dynamic programming algorithm.

FIG. 4 depicts a schematic view elucidating the concept of local alignment using the dynamic programming algorithm.

FIG. 5 shows the double dynamic programming algorithm.

FIG. 6 shows an efficient process for carrying out the local structural alignment method demonstrated by Taylor and Orengo.

FIG. 7 shows circular permutation.

FIG. 8 shows glucanase as an example for circular permutation.

FIG. 9 depicts structural differences in the structural environment between two proteins having a relationship of circular permutation.

FIG. 10 shows the concept governing the reconstruction of structural environments relating to the present invention.

FIG. 11 depicts a block diagram of the hardware used to obtain the structural local alignments employing the double dynamic programming algorithm relating to the present invention.

FIG. 12 shows a list of proteins with similar local structures that were used for computation to compare the conventional and the reconstructed structural environments relating to the present invention.

FIG. 13 shows structural local alignments between HIV-1 protease and aspartic proteinase for comparison of structural local alignments resulting from conventional and reconstructed structural environments.

FIG. 14 shows the results of structural local alignment between HIV-1 protease and aspartic proteinase under various cut-off distances and ΔN cut-off values.

FIG. 15 shows the CPU time required to obtain structural local alignments between HIV-1 protease and aspartic proteinase under various cut-off distances and ΔN cut-off values.

FIG. 16 shows structural local alignments between cyclooxygenase and E-selectin.

FIG. 17 shows the results of structural local alignment between cyclooxygenase and E-selectin under various cut-off distances and ΔN cut-off values.

FIG. 18 shows the CPU time required to obtain structural local alignments between cyclooxygenase and E-selectin under various cut-off distances and ΔN cut-off values.

FIG. 19 shows structural local alignments between immunoglobulin Fab domains.

FIG. 20 shows structural local alignments between immunoglobulin Fab domains under various cut-off distances and ΔN cut-off values.

FIG. 21 shows the CPU time required to obtain structural local alignments between immunoglobulin Fab domains under various cut-off distances and ΔN cut-off values.

FIG. 22 shows the names of the circular permuted proteins and symbols in a protein databank used for the tests of our methodology.

FIG. 23 shows the results of structural local alignment between β-glucanase and circular permuted glucanase without the approximation of cut-off distance and ΔN cut-off value.

FIG. 24 shows the results of structural local alignment between β-glucanase and circular permuted glucanase. The approximations of a cut-off distance of 20 Å and a ΔN cut-off value of 20 were used in this calculation.

FIG. 25 shows structural local alignments between β-glucanase and circular permuted glucanase under various cut-off distances and ΔN cut-off values.

FIG. 26 shows the CPU time required to obtain structural local alignments between β-glucanase and circular permuted glucanase under various cut-off distances and ΔN cut-off values.

FIG. 27 shows structural local alignments between avidin and circular permuted streptavidin without the approximations of distance cut-off and ΔN cut-off.

FIG. 28 shows structural local alignments between avidin and circular permuted streptavidin where the cut-off distance was 20 Å and the ΔN cut-off value was 20.

FIG. 29 shows the results of structural local alignment between avidin and circular permuted streptavidin under various cut-off distances and ΔN cut-off values.

FIG. 30 shows the CPU time required to obtain structural local alignments between avidin and circular permuted streptavidin under various cut-off distances and ΔN cut-off values.

FIG. 31 shows the results of structural local alignment between phthalate dioxygenase reductase and FMN-binding protein without the approximations of distance cut-off and ΔN cut-off.

FIG. 32 shows the results of structural local alignment between phthalate dioxygenase reductase and FMN-binding protein under a cut-off distance of 15 Å and a ΔN cut-off value of 50.

FIG. 33 shows the results of structural local alignment between phthalate dioxygenase reductase and FMN-binding protein under various cut-off distances and ΔN cut-off values.

FIG. 34 shows the CPU time required to obtain structural local alignments between phthalate dioxygenase reductase and FMN-binding protein under various cut-off distances and ΔN cut-off values.

FIG. 35 shows the results of structural local alignment between human glutathione synthetase and Escherichia coli glutathione synthetase without the approximations of distance cut-off and ΔN cut-off.

FIG. 36 shows the results of structural local alignment between human glutathione synthetase and Escherichia coli glutathione synthetase where the cut-off distance was 50 Å and no consideration was given to ΔN cut-off.

FIG. 37 shows the results of structural local alignment between human glutathione synthetase and Escherichia coli glutathione synthetase under various cut-off distances and ΔN cut-off values.

FIG. 38 shows the CPU time required to obtain structural local alignments between human glutathione synthetase and Escherichia coli glutathione synthetase under various cut-off distances and ΔN cut-off values.

FIG. 39 shows the results of structural local alignment between transaldolase and fructose-1,6-bisphosphatealdolase without the approximations of distance cut-off and ΔN cut-off.

FIG. 40 shows the results of structural local alignment between transaldolase and fructose-1,6-bisphosphatealdolase where the cut-off distance was 20 Å and the ΔN cut-off value was 20.

FIG. 41 shows the results of structural local alignment between transaldolase and fructose-1,6-bisphosphatealdolase under various cut-off distances and ΔN cut-off values.

FIG. 42 shows the CPU time required to obtain structural local alignments between transaldolase and fructose-1,6-bisphosphatealdolase under various cut-off distances and ΔN cut-off values.

DESCRIPTION OF THE PREFERRED EMBODIMENTS

The embodiments of the present invention will next be described in detail with reference to the drawings. First, the structural local alignment method of the present invention that uses the double dynamic programming algorithm will be described.

Here, we considered that the difficulty in obtaining structural local alignments of circular permuted proteins resides in the fact that because vector components are lined up in order of residue No. from the N-terminal residue of a protein to the C-terminal residue when structural environments are constructed, the structural environments become dependent on order of residue upon the protein sequences. Therefore, we have attempted to reconstruct structural environments which will be robust against circular permutation, as described below.

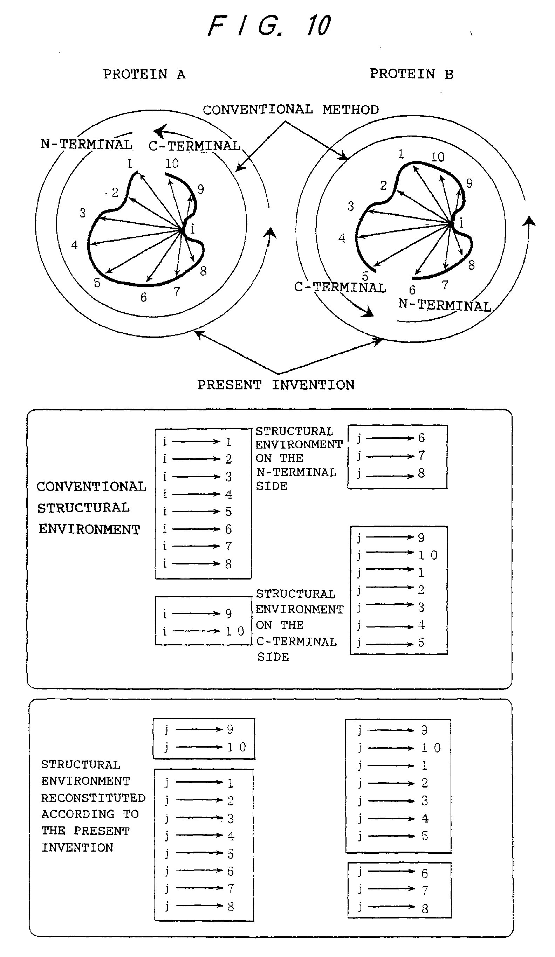

> (1) To obtain the structural environment for residue i, vectors for the N-terminal side against residue i and those for the C-terminal side against residue i are obtained separately.
> (2) As shown in FIG. 10, vectors for the C-terminal side against residue i are aligned in order of residue No.; and subsequently vectors for the N-terminal side against residue i are aligned in order of residue No.

**[0076]** Using FIG. 10, the conventional method and the method of the present invention are described in detail below.

In the conventional method, structural environment components for residue i in protein A are placed in the order of 1, 2, 3, 4, 5, 6, 7, 8, 9, and 10, whereas in the method of the present invention they are 9, 10, 1, 2, 3, 4, 5, 6, 7, and 8. In contrast, those for residue i in protein B are placed in the order of 6, 7, 8, 9, 10, 1, 2, 3, 4, and 5 in the conventional method whereas those that are not affected by the interchange between the N-terminal and C-terminal sequences are lined up in the order of 9, 10, 1, 2, 3, 4, 5, 6, 7, and 8 in the method of the present invention. Performing such an operation, as shown in FIG. 10, we could construct an environment not affected by the interchange between the N-terminal and C-terminal sequences.

To obtain two or more structural local alignments efficiently, upon backtracking on an upper level DP matrix, Taylor and Orengo put zero against the values of residue pairs surrounding the path that has been already used, thereby leading to effective finding of a new local structural alignment in a different region (see FIG. 5).

However, when very similar structures were present, there were cases in which this method was unable to find the other similar structures. In other words, high score values are given to residue pairs between very similar structures. In the case of structural local alignment, it is preferred to put zero or a value close to zero as an extension gap penalty; however, if this is the case, the gap penalty, a + b*(K - 1) where b∼0, is given (see FIG. 1); and thus, even when the gap becomes longer, the penalty value does not become larger. Thus, if very similar structures are present, a very high score value $D_{x,y}$ is given to a residue pair x, y in the similar structure; and then, almost without attenuation, the value is introduced to the recurrence equation as the E or F value for the term relating to the extension gap. In this case, either $E_{i,j}$ or $F_{i,j}$ is chosen because of the relationship of $D_{x,y} - [a + b \times (k - 1)] > S_{i,j} + D_{i-1,j-1}$, even if there are residue pairs with relatively high $S_{i,j}$ values. As a consequence, the path is merged with the alignment detected previously by the gap.

On the other hand, upon structural local alignment, the present invention resets Si, j values of residue pairs surrounding the backtracked residue pairs; i. e., to perform upper level DP, the values at the corresponding residue pairs on a matrix (LMtx) are reset to atypical values not usually found (very small negative values). Then, for the subsequent alignment, upper level DP is carried out disregarding the region.

In this way, by avoiding the generation of paths from the residue pairs which have already been selected for the alignment, the paths that could converge into the alignment previously found are all eliminated to allow re-computation of upper level PD. Thus, with no influence from the score values in the structural local alignment previously detected, a new alignment can now be detected by using the present invention.

In addition, the present program is also designed to incorporate a condition (distance cut-off condition) that spatially restricts component residues for a structural environment in order to speed up computation, and also a condition (ΔN cut-off condition) that sets a restriction on the difference of the number of the components between two structural environments. (Toh H, CABIOS (1997), 396, 13, 387)

Cutting off distance is an approximation such that the environment of each amino acid residue is expressed by residue positions relative to only other residues within a sphere with a radius of the cut-off distance. On the other hand, ΔN cut-off is an approximation based on the assumption that residue numbers in each structural environment are similar when the local structures of compared residues are similar to one another. In turn, this leads to the assumption that the local structures are not similar when the difference in their residue numbers is larger than the cut-off value. If this is the case, then the computation of their similarity will be omitted. Further, the program is also able to introduce a penalty either to avoid paired alignments between two structures with different secondary structures, such as an α-helix and a β-strand or to make it difficult to insert a gap within such secondary structures.

The results of actual structural local alignments are shown below.

Firstly, as a computing example of a structural local alignment that can be also accurately obtained by the conventional method, structural local alignments of proteins were carried out in the cases of proteins not involved in circular permutation and not similar in overall tertiary structure but locally similar.

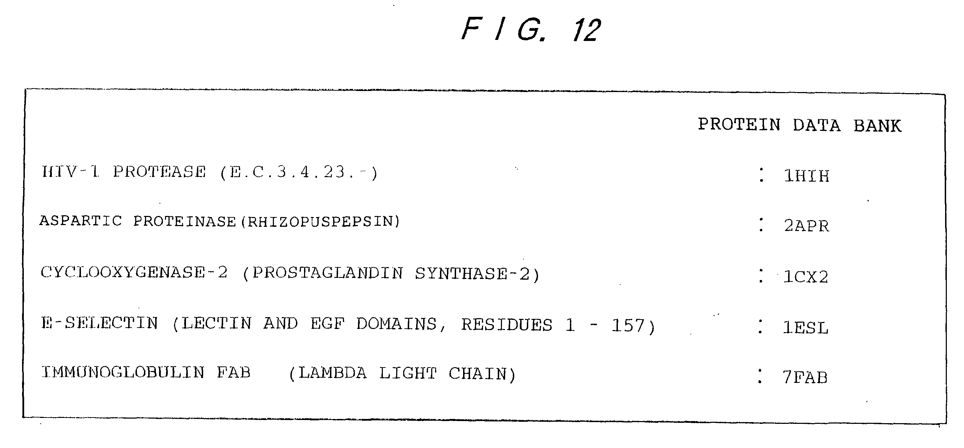

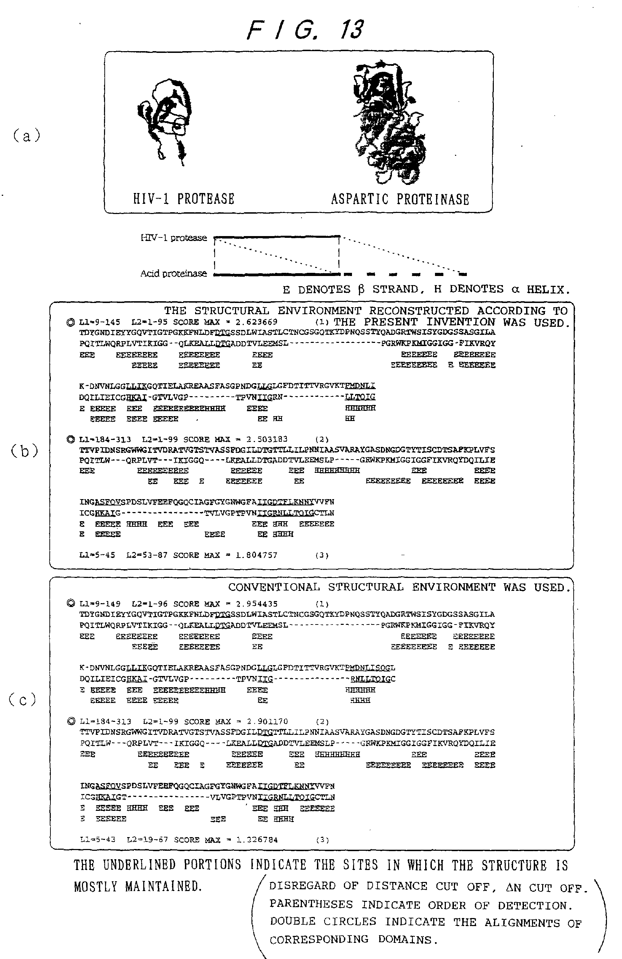

FIG. 12 shows a list of proteins with similar local structures used for computation to compare conventional and reconstructed structural environments using the present invention. The list also shows the names of the proteins and their protein databank symbols. The results of structural local alignment of the proteins described in FIG. 12 are shown in FIGs. 13-21 that compare the conventional and the reconstructed structural environments relating to the present invention.

FIG. 13 shows structural local alignments of HIV-1 protease and aspartic proteinase. Here, among the alignments obtained, the top two high score alignments are shown. FIG. 13(a) shows tertiary structures of HIV-1 protease and aspartic proteinase, and FIG. 13 (b) shows an example of structural local alignments where structural environments were reconstructed using the present invention. FIG. 13(c) shows the alignment example using conventional structural environments.

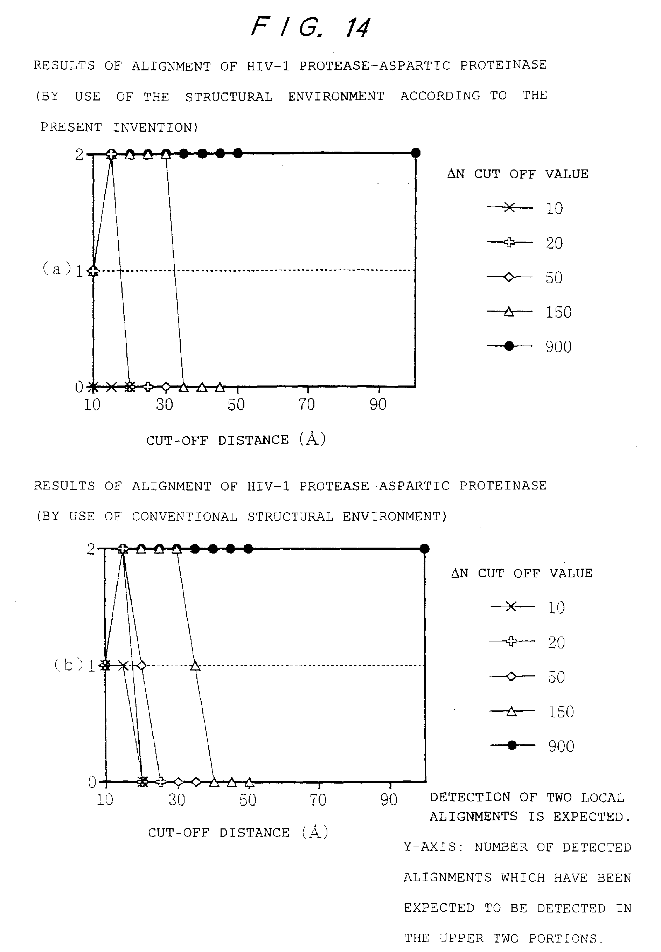

FIG. 14 shows the results of structural local alignment of HIV-1 protease and aspartic proteinase under various cut-off distances and ΔN cut-off values. FIG. 14(a) shows an example applying the reconstructed structural environments related to the present invention, whereas FIG. 14(b) shows an example using conventional structural environments.

Aspartic proteinase is constituted by two domains whose structures are similar to the HIV-1 protease. Thus, it is expected that, upon structural local alignment, two structural local alignments will be obtained. The vertical axis shows the domain numbers detected.

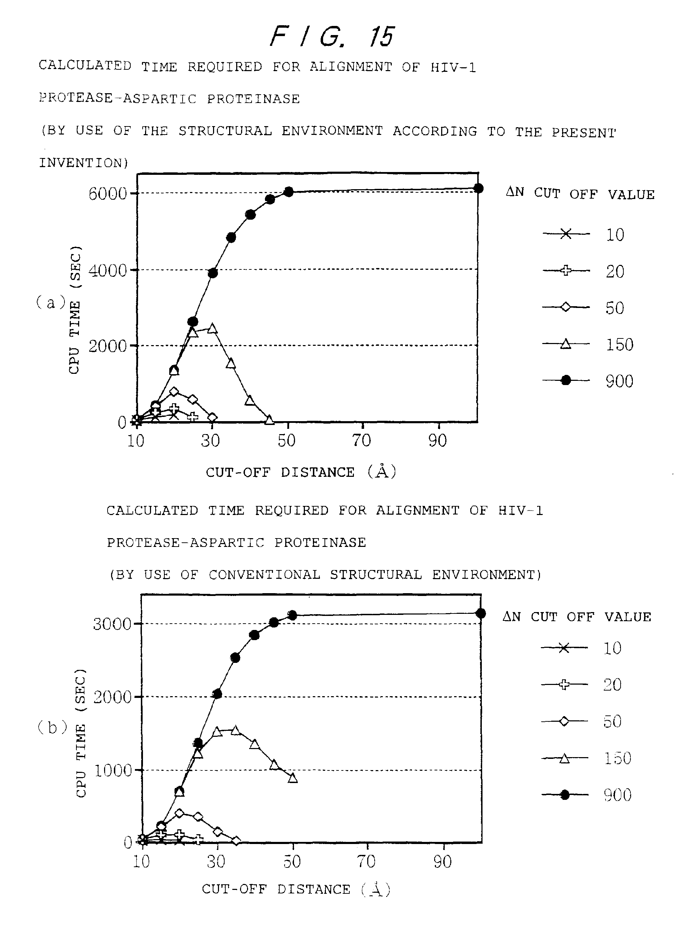

Further, FIG. 15 shows the CPU time required to obtain the structural local alignments of HIV-1 protease and aspartic proteinase under the approximations of various cut-off distances and ΔN cut-off values. FIG. 15(a) shows an example where the structural environments were reconstructed using the present invention, whereas FIG. 15(b) shows an example using the conventional structural environments.

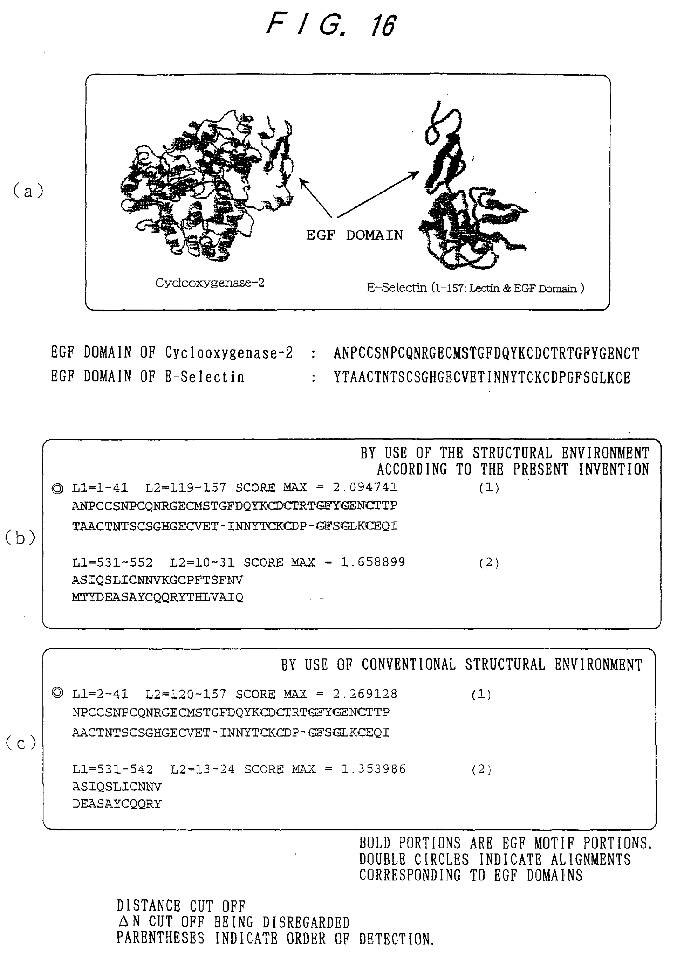

FIG. 16 shows structural local alignments of cyclooxygenase and E-selectin. FIG. 16(a) shows tertiary structures of cyclooxygenase and E-selectin. FIG. 16(b) shows an example applying the reconstructed structural environments of the present invention. FIG. 16(c) shows an example using conventional structural environments.

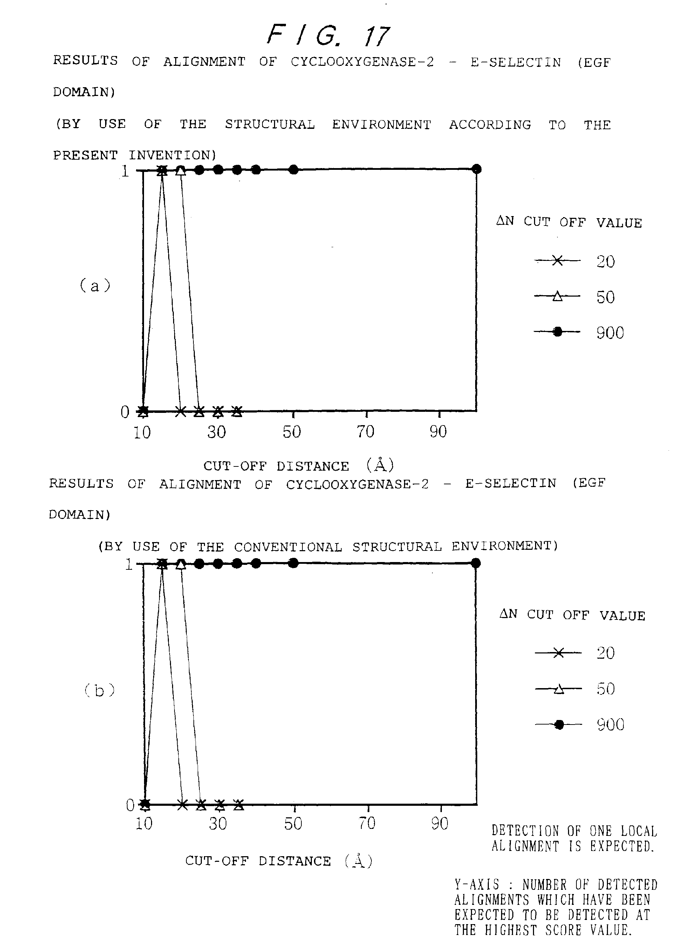

FIG. 17 shows the results of structural local alignment of cyclooxygenase and E-selectin under various cut-off distances and ΔN cut-off values. FIG. 17(a) shows structural local alignment results using the reconstructed structural environments of the present invention; and FIG. 17(b) shows alignment results using conventional structural environments.

Each protein has one EGF domain, and thus it is expected that, upon structural local alignment, one structural local alignment will be obtained. The vertical axis shows the domain numbers detected.

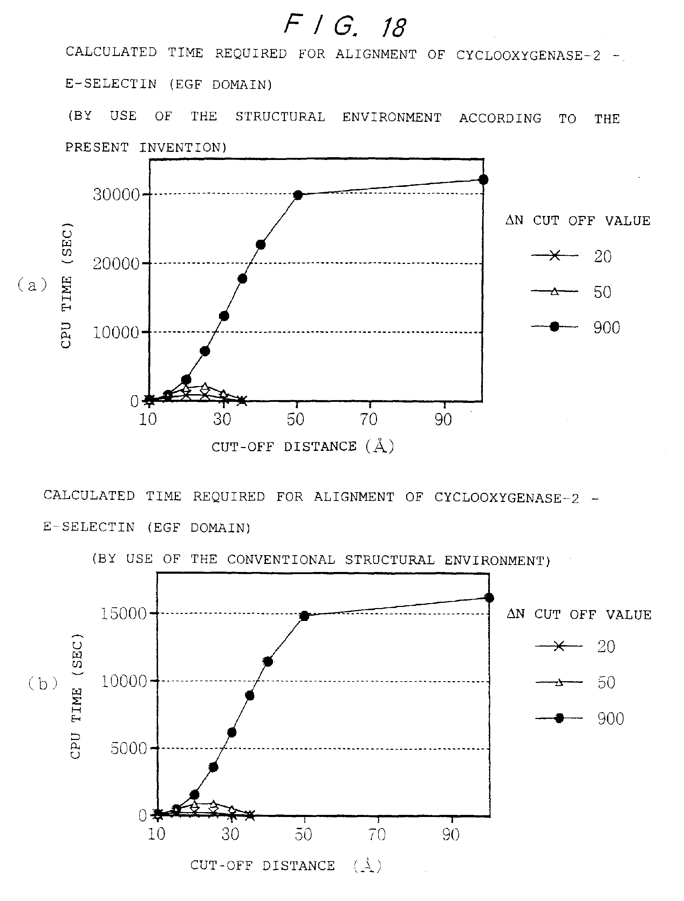

FIG. 18 shows the CPU time required to obtain the structural local alignments between cyclooxygenase and E-selectin under various cut-off distances and ΔN cut-off values. FIG. 18(a) shows the CPU time required to obtain the structural local alignments when the structural environments were reconstructed using the present invention; and FIG. 18(b) shows the CPU time required to obtain the structural local alignments using conventional structural environments.

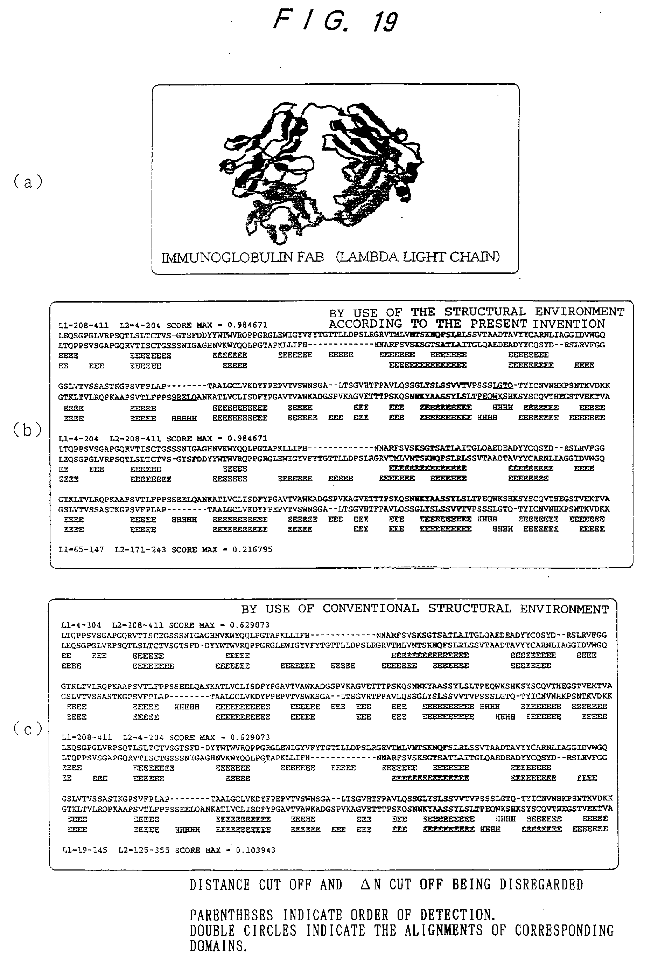

FIG. 19 shows structural local alignments between immunoglobulin Fab domains. Since in this case the same proteins were compared, a perfectly matched structural alignment over the entire sequence would represent the maximum score. Omitting this alignment, the other two structural alignments obtained are shown. FIG. 19(a) shows the tertiary structure of immunoglobulin Fab domains, and FIG. 19(b) shows an example using the reconstructed structural environments in the present invention; and FIG. 19(c) shows an example using conventional structural environments.

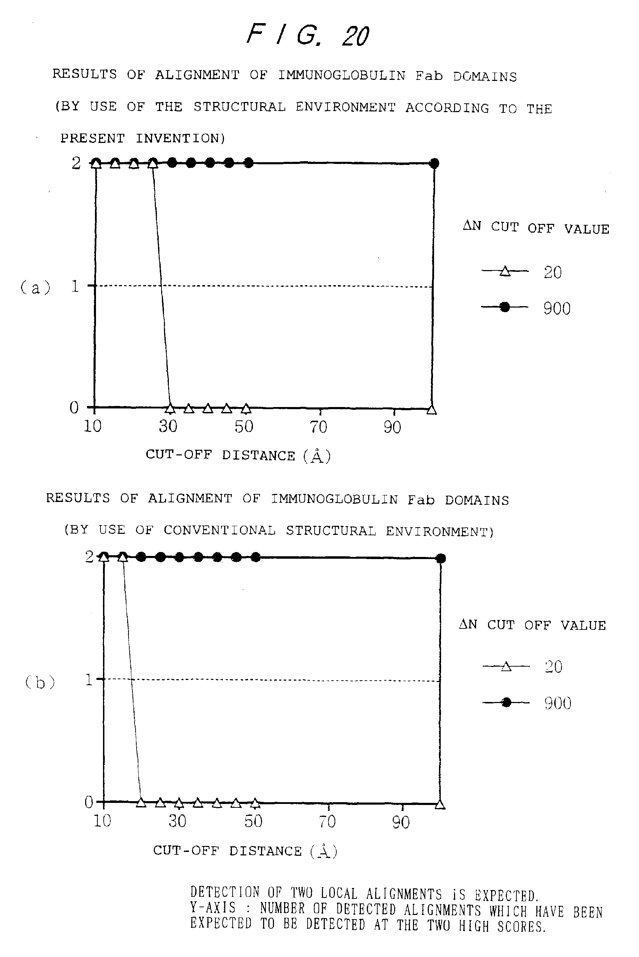

FIG. 20 shows the results of structural local alignment between immunoglobulin Fab domains under various cut-off distances and ΔN cut-off values. FIG. 20 (a) shows the results of structural local alignment obtained by the application of the structural environments reconstructed according to the present invention; and FIG. 20(b) shows the alignment results using conventional structural environments.

Because two similar structures are present in one molecule, two structural local alignments are expected to be obtained. The vertical axis shows domain numbers detected.

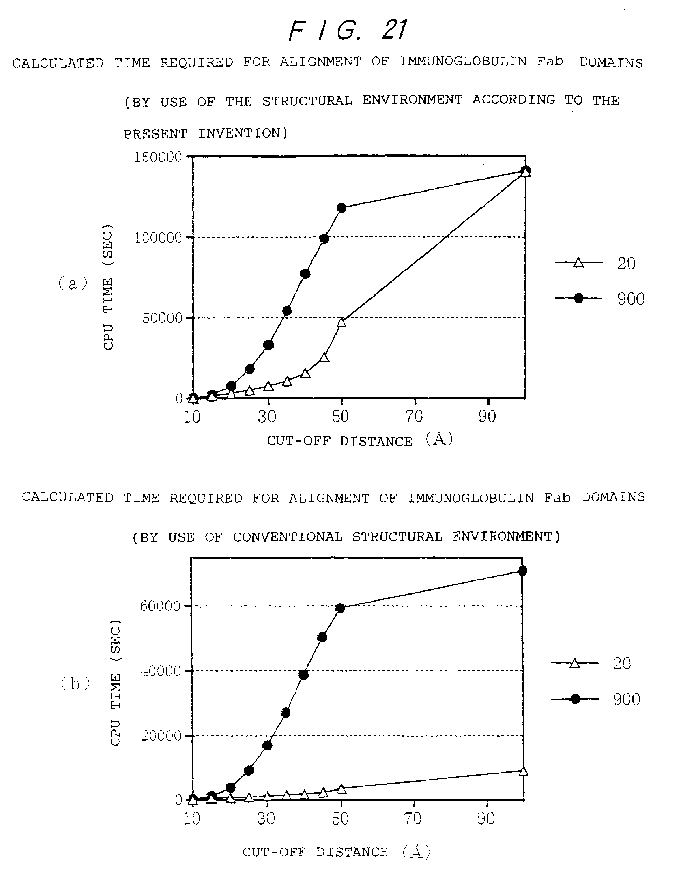

FIG. 21 shows the CPU time required to obtain the structural local alignments between immunoglobulin Fab domains under various cut-off distances and ΔN cut-off values. FIG. 21(a) shows the CPU time required to obtain the structural local alignments using the reconstructed structural environments according to the present invention; and FIG. 21(b) shows the CPU time required to obtain the structural local alignments using conventional structural environments.

As shown in FIGs. 13-21, in the case of proteins having no relationship of circular permutation, there was not much difference in the structural local alignments between the conventional method and the method of the present invention, indicating that, applying the reconstructed structural environments of the present invention, accurate structural local alignments can be obtained even for the proteins having no relationship of circular permutation. In the case of either the conventional structural environment or the reconstructed structural environment of the present invention, among the approximation methods of cut-off distance and ΔN cut-off in order to speed up computation, the distance cut-off approximation method worked very efficiently for structural local alignment so that, when the cut-off distance was 10-20 Å, the CPU time was greatly shortened without loss in alignment accuracy.

However, upon introduction of ΔN cut-off, alignment results were greatly affected by the setting of cut-off distance values, indicating that the ΔN cut-off approximation method is not suitable for structural local alignment probably due to the following reason. In the case in which similar structures are restricted locally, structural environments constituted by only nearby local structures would be constructed under the condition of a small cut-off distance, and thus residue numbers constituting the structural environments at similar structural regions could virtually coincide. However, if the cut-off distance is large, even though their nearby local structures are similar, the structural environments of residues at the similar regions would be constructed considering those at distant non-similar regions, thus resulting in a great difference in their component vector numbers.

Consequently, due to the ΔN cut-off setting, those structural regions are judged to be dissimilar from the beginning, and thereby the lower-level DP computation is omitted.

Thus, from the above alignment results, it has become clear that regarding the structural local alignments of proteins not involved in circular permutation, the structural environments reconstructed according to the present invention provide results of almost the same level of accuracy as compared to that obtained through conventional structural environments.

Next, remarkable effects on structural local alignments that are obtained by using reconstructed structural environments relating to the present invention will be described using examples of proteins with the relationship of circular permutation.

To avoid the dependency of the sequence length of alignment score values, the alignment score value was defined as one obtained by dividing each score by a sequence length; then, the alignments sorted in order of value from the highest were regarded as the alignment results. Concerning interpretation of the obtained results, when one sequence related to circular permutation was detected with the maximum score, it was judged that one alignment related to circular permutation had been detected. Then, when the other sequence related to circular permutation was detected with the second highest score, it was judged that both sequences related to circular permutation had been obtained.

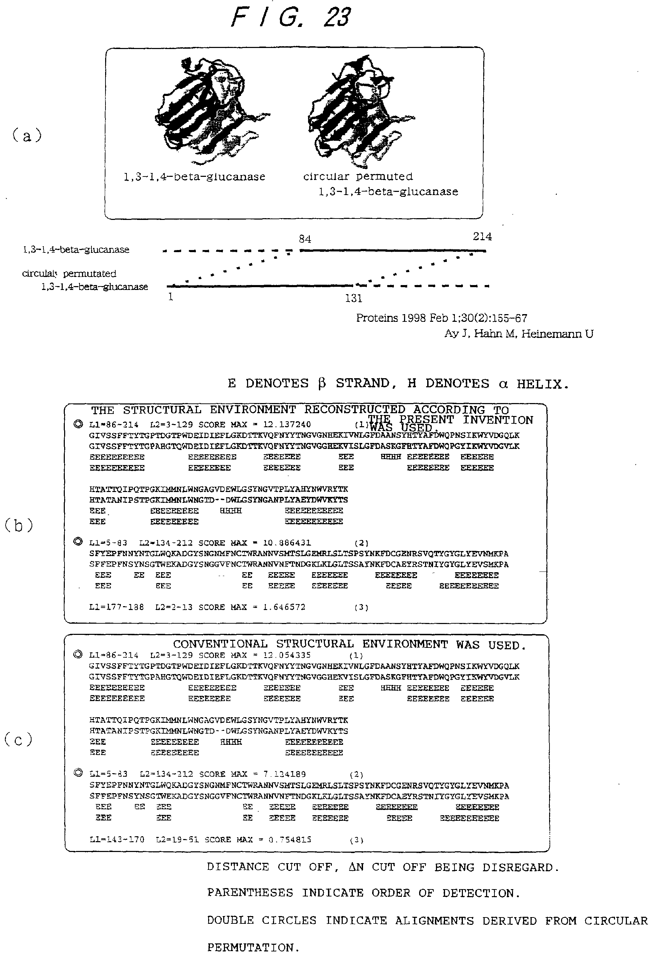

FIGs. 23-42 show comparisons between the resultant structural local alignments obtained by using structural environments reconstructed according to the present invention and those obtained by using conventional structural environments.

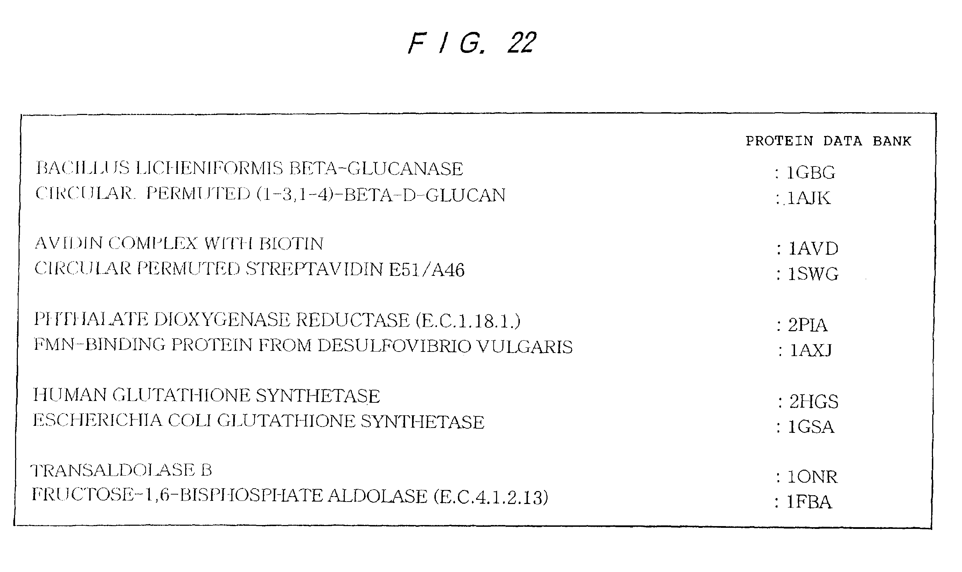

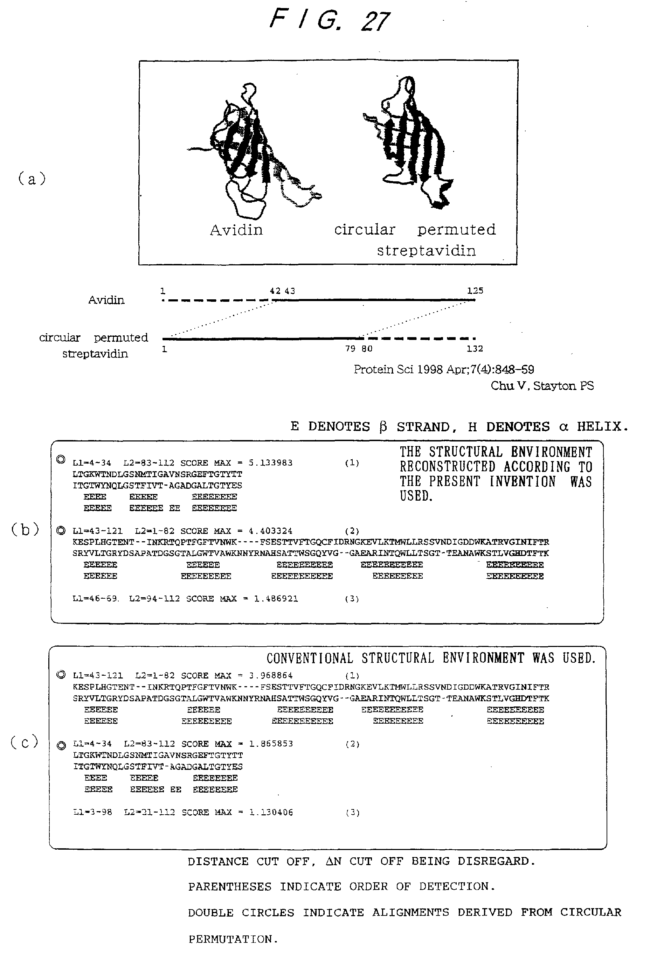

FIG. 22 shows a list of proteins used for computing structural local alignments. FIGs. 23-26 and FIGs. 27-30 show proteins artificially synthesized to have the circular permuted relationship, wherein their tertiary structures were determined by X-ray crystal analysis (for FIGs. 23-26, Proteins 1998 Feb. 1; 30 (2): 155-167; and for FIGs. 27-30, Protein Sci. 1998 Apr. 7 (4): 848-859).

FIGs. 23 and 27 show structural local alignments without the setting of cut-off distance and ΔN cut-off. Each alignment was arranged in order of upper-level DP score values from the highest; then each score was divided by an alignment sequence length to avoid dependency of the sequence length of score values, and then rearranged in order of value from the highest; and thus the obtained alignments were regarded as the alignment results.

Each FIG. will be explained in detail below.

FIG. 23 shows the results of structural local alignment of β-glucanase and circular permuted glucanase under no cut-off distance and no ΔN cut-off. FIG. 23(a) shows tertiary structures of β-glucanase and circular permuted glucanase. FIG. 23(b) shows the alignment results obtained by using structural environments reconstructed according to the present invention. FIG. 23(c) shows the alignment results obtained by using conventional structural environments.

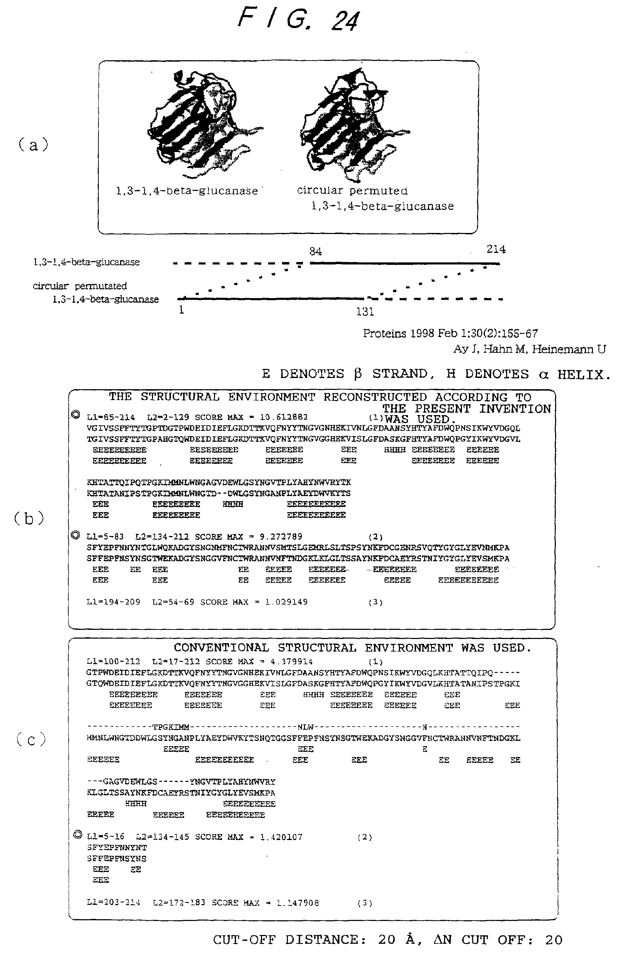

FIG. 24 shows the alignment results of β-glucanase and circular permuted glucanase under a cut-off distance of 20 Å and a ΔN cut-off of 20. FIG. 24(a) shows the tertiary structures of β-glucanase and circular permuted glucanase. FIG. 24(b) shows the alignment results obtained by using structural environments reconstructed according to the present invention. FIG. 24 (c) shows the alignment results using conventional structural environments.

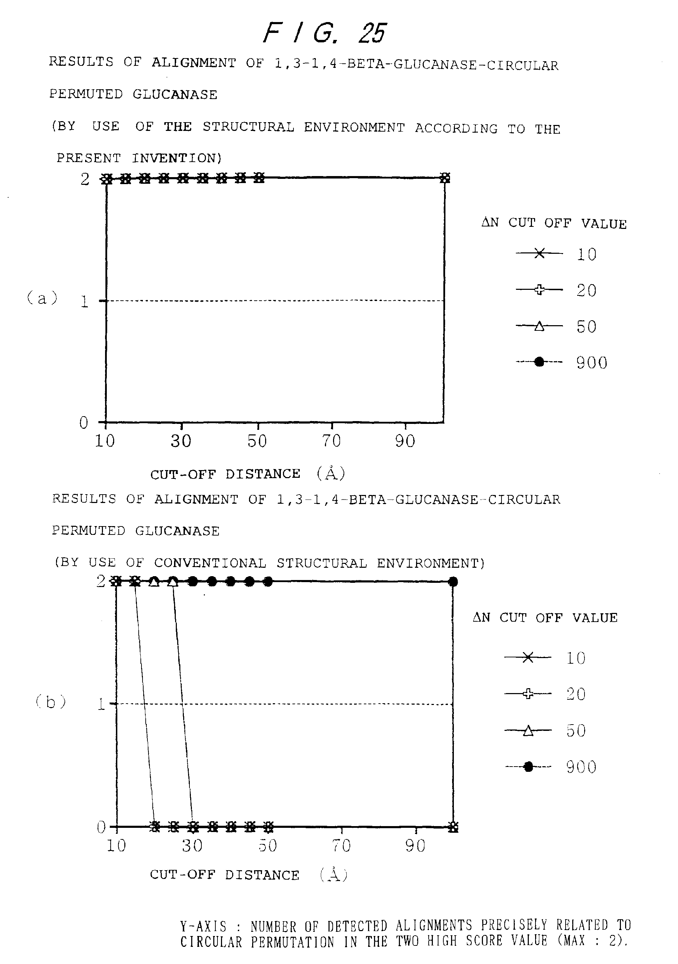

FIG. 25 shows the structural local alignments of β-glucanase and circular permuted glucanase under various cut-off distances and ΔN cut-off values. The vertical axis shows the alignment number detected for circular permutation. FIG. 25(a) shows the alignment results obtained by using structural environments reconstructed according to the present invention. FIG. 25(b) shows the alignment results obtained by using conventional structural environments.

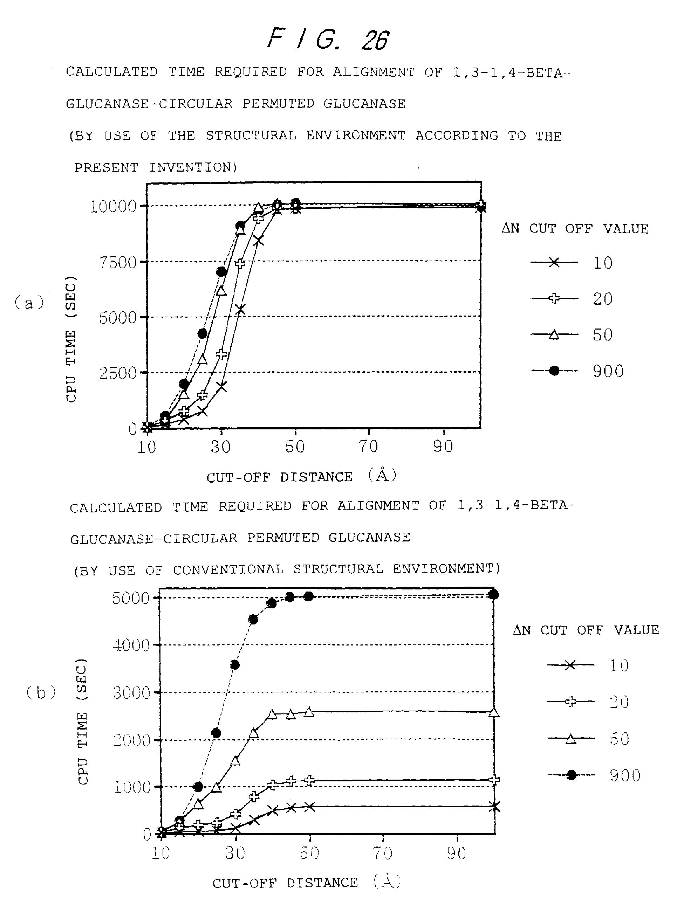

FIG. 26 shows the CPU time required to obtain the structural local alignments of β-glucanase and circular permuted glucanase under various cut-off distances and ΔN cut-off values. FIG. 26(a) shows the CPU time required to obtain the structural local alignments using structural environments reconstructed according to the present invention. FIG. 26(b) shows the CPU time required to obtain the structural local alignments using conventional structural environments.

FIG. 27 shows the results of structural local alignment of avidin and circular permuted streptavidin where no consideration was given to cut-off distance and ΔN cut-off. FIG. 27(a) shows the tertiary structures of avidin and circular permuted streptavidin. FIG. 27(b) shows the alignment results obtained by using structural environments reconstructed according to the present invention. FIG. 27(c) shows the alignment results obtained by using conventional structural environments.

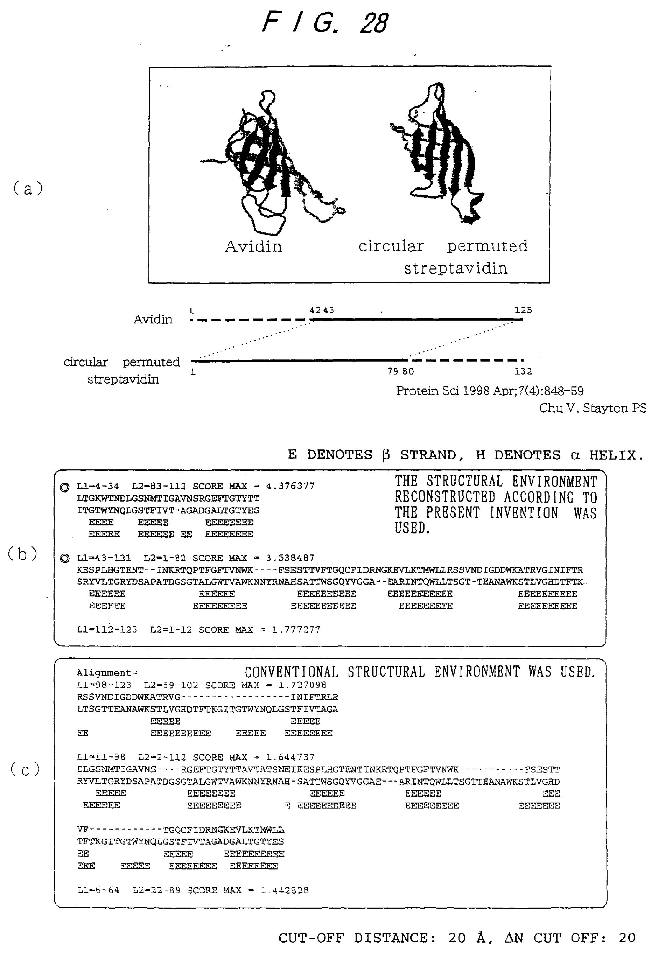

FIG. 28 shows the results of structural local alignment of avidin and circular permuted streptavidin under a cut-off distance of 20 Å and a ΔN cut-off value of 20. FIG. 28(a) shows tertiary structures of avidin and circular permuted streptavidin. FIG. 28(b) shows the alignment results obtained by using structural environments reconstructed according to the present invention. FIG. 28(c) shows the alignment results obtained by using conventional structural environments.

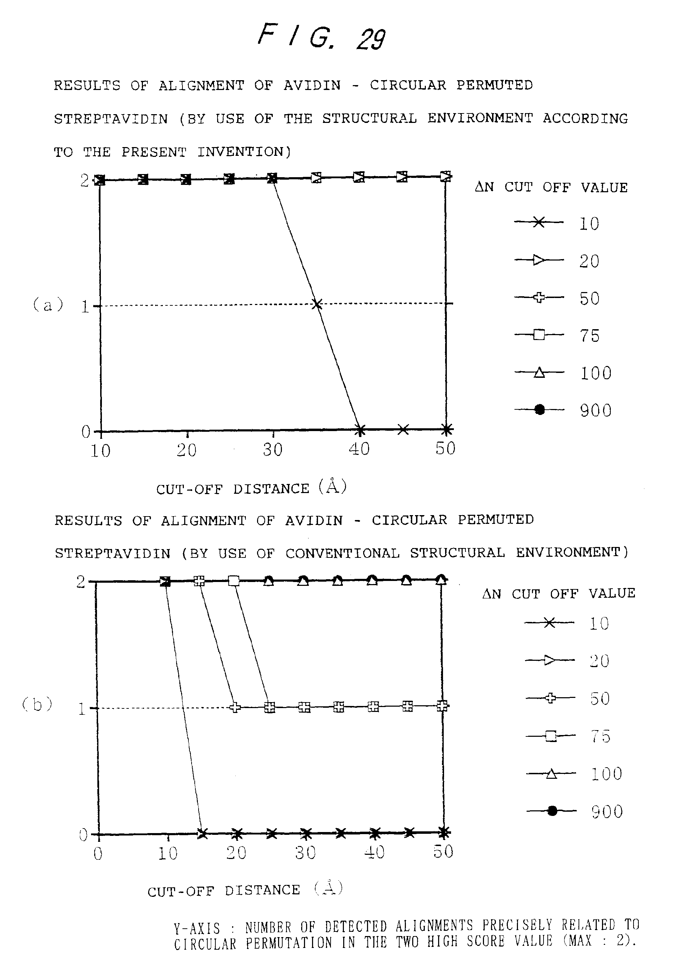

FIG. 29 shows the results of structural local alignment of avidin and circular permuted streptavidin under various cut-off distances and ΔN cut-off values. FIG. 29(a) shows the alignment results obtained by using structural environments reconstructed according to the present invention. FIG. 29(b) shows the alignment results obtained by using conventional structural environments.

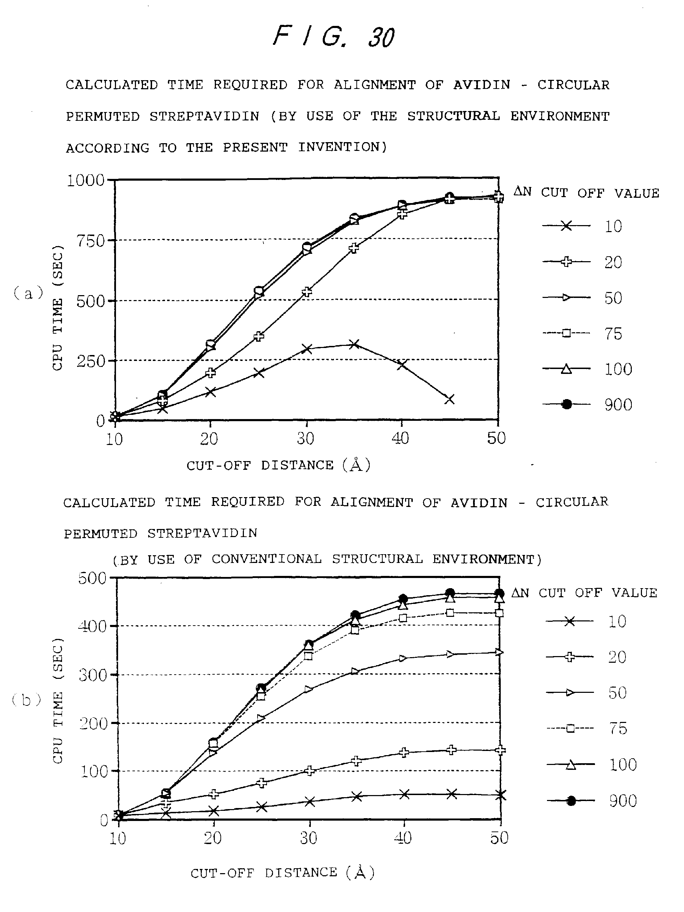

FIG. 30 shows the CPU time required to obtain the structural local alignments of avidin and circular permuted streptavidin under various cut-off distances and ΔN cut-off values. FIG. 30(a) shows the CPU time required to obtain the structural local alignments using the structural environments reconstructed according to the present invention. FIG. 30(b) shows the CPU time required to obtain the alignments using conventional structural environments.

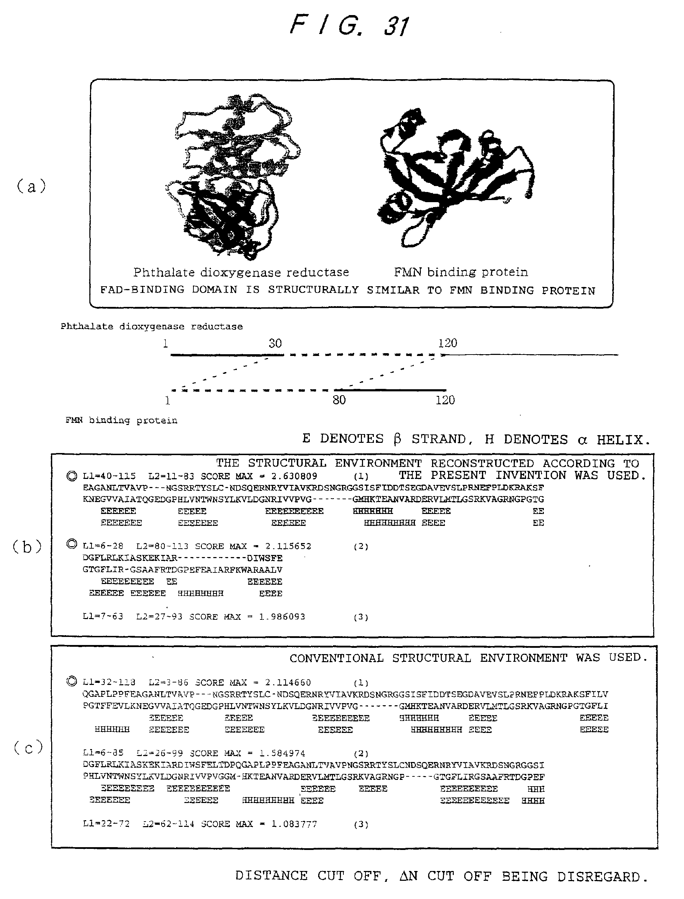

FIGs. 31-34 show the results of structural local alignment of phthalate dioxygenase reductase and FMN-binding protein.

FIG. 31 shows the structural local alignments of phthalate dioxygenase reductase and FMN-binding protein under no cut-off distance and no ΔN cut-off. FIG. 31(a) shows the tertiary structures of phthalate dioxygenase reductase and FMN-binding protein. FIG. 31(b) shows alignment results obtained by using structural environments reconstructed according to the present invention. FIG. 31(c) shows the alignment results obtained by using conventional structural environments.

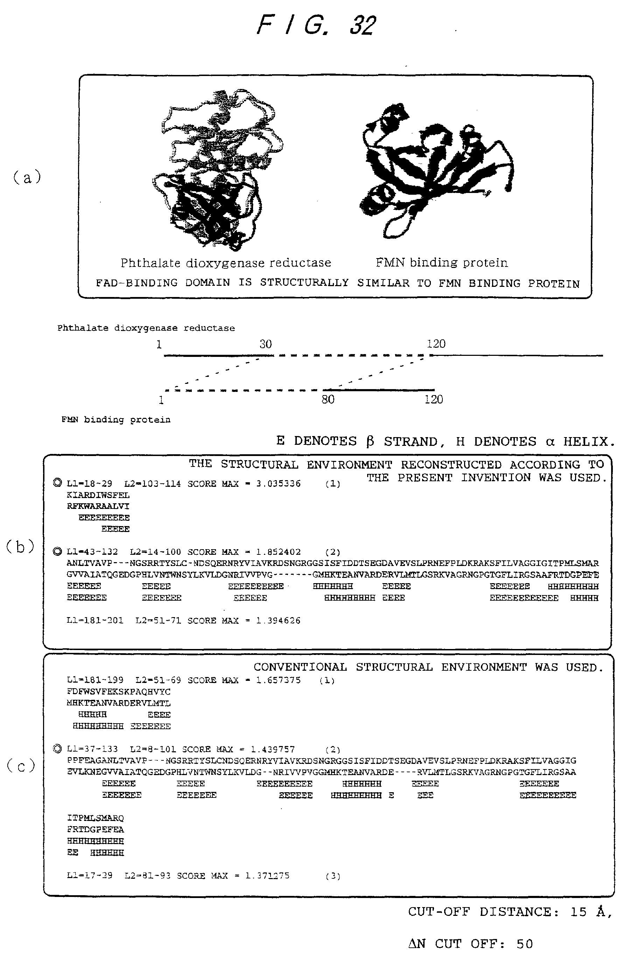

FIG. 32 shows the structural local alignments of phthalate dioxygenase reductase and FMN-binding protein under a cut-off distance of 15 Å and a ΔN cut-off of 50. FIG. 32(a) shows the tertiary structures of phthalate dioxygenase reductase and FMN-binding protein. FIG. 32(b) shows alignment results obtained by using structural environments reconstructed according to the present invention. FIG. 32(c) shows the alignment results obtained by using conventional structural environments.

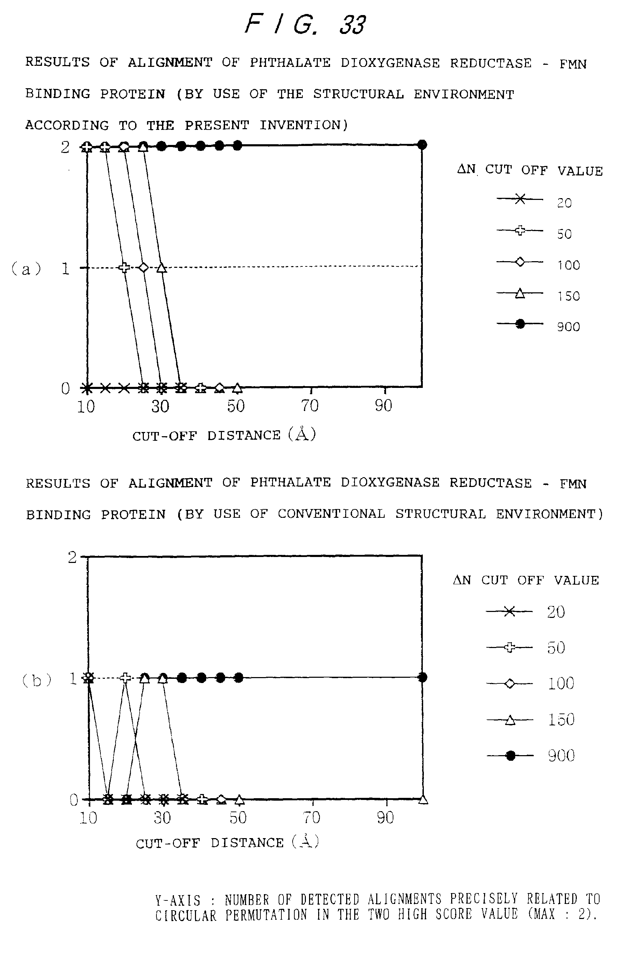

FIG. 33 shows the results of structural local alignment of phthalate dioxygenase reductase and FMN-binding protein under various cut-off distances and ΔN cut-off values. FIG. 33(a) shows the alignment results obtained by using structural environments reconstructed according to the present invention. FIG. 33 (b) shows the alignment results obtained by using conventional structural environments.

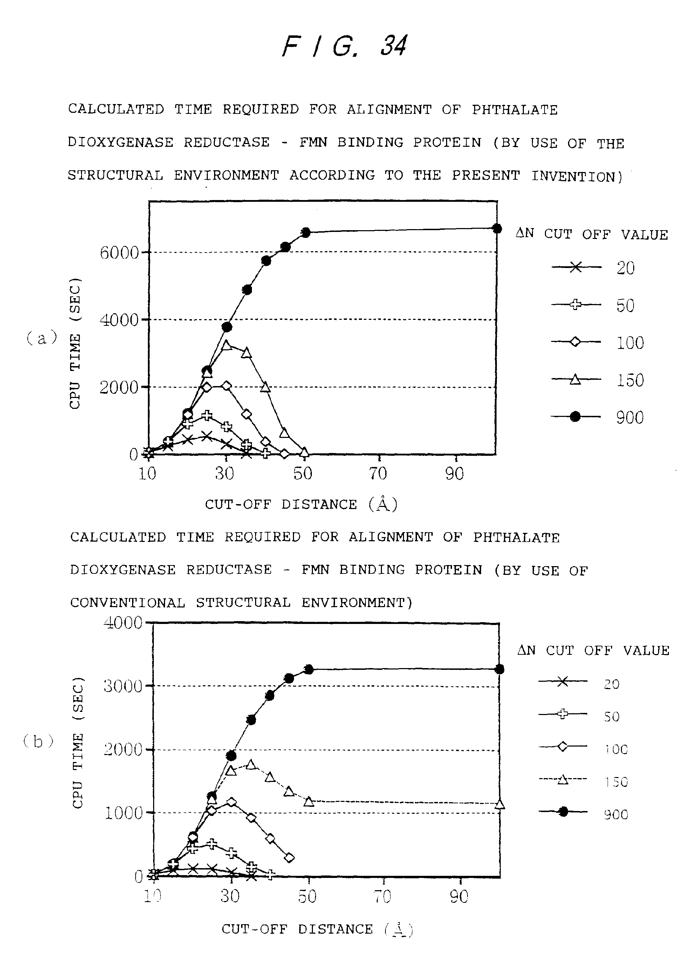

FIG. 34 shows the CPU time required to obtain the structural local alignment of phthalate dioxygenase reductase and FMN-binding protein under various cut-off distances and ΔN cut-off values. FIG. 34(a) shows the CPU time required to obtain the structural local alignments using the structural environments reconstructed according to the present invention. FIG. 34(b) shows the CPU time required to obtain the structural local alignments using conventional structural environments.

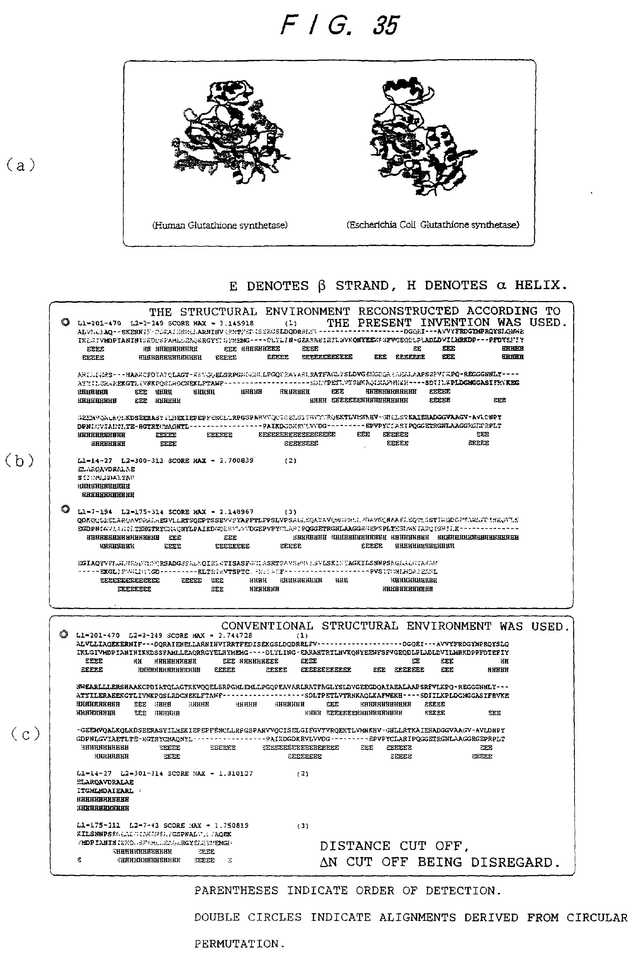

FIG. 35 shows the results of structural local alignment of human glutathione synthetase and Escherichia coli glutathione synthetase with no consideration given to cut-off distance and ΔN cut-off. FIG. 35(a) shows tertiary structures of human glutathione synthetase and Escherichia coli glutathione synthetase. FIG. 35(b) shows an example obtained by using structural environments reconstructed according to the present invention. FIG. 35(c) shows an example obtained by using conventional structural environments.

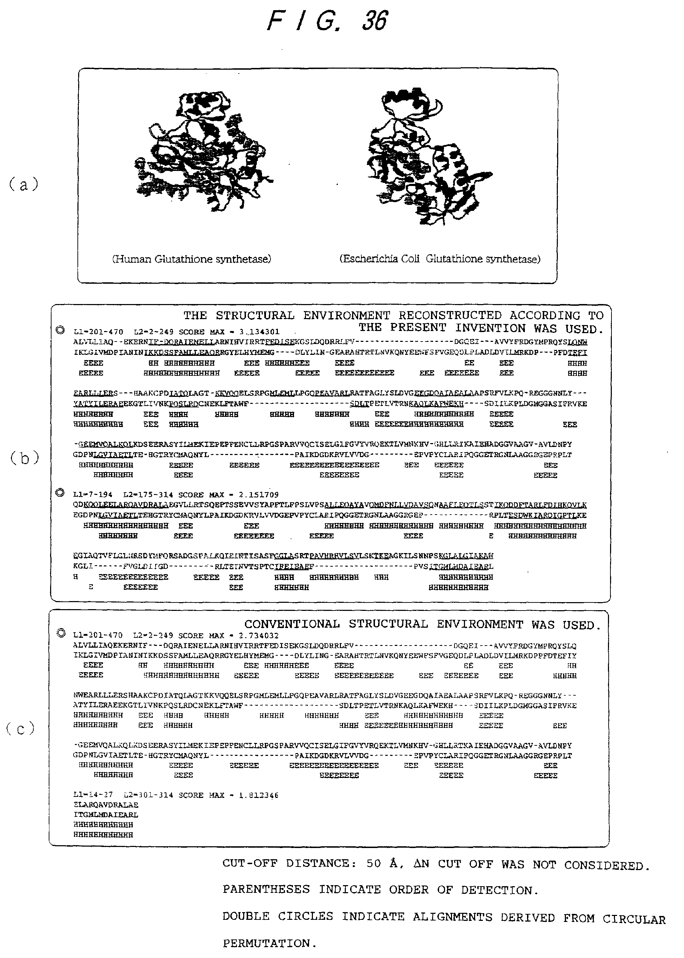

FIG. 36 shows the results of structural local alignment of human glutathione synthetase and Escherichia coli glutathione synthetase under a cut-off distance of 50 Å and no ΔN cut-off value. FIG. 36(a) shows human glutathione synthetase and Escherichia coli glutathione synthetase. FIG. 36(b) shows results obtained by using structural environments reconstructed according to the present invention. FIG. 36(c) shows the alignment results obtained by using conventional structural environments.

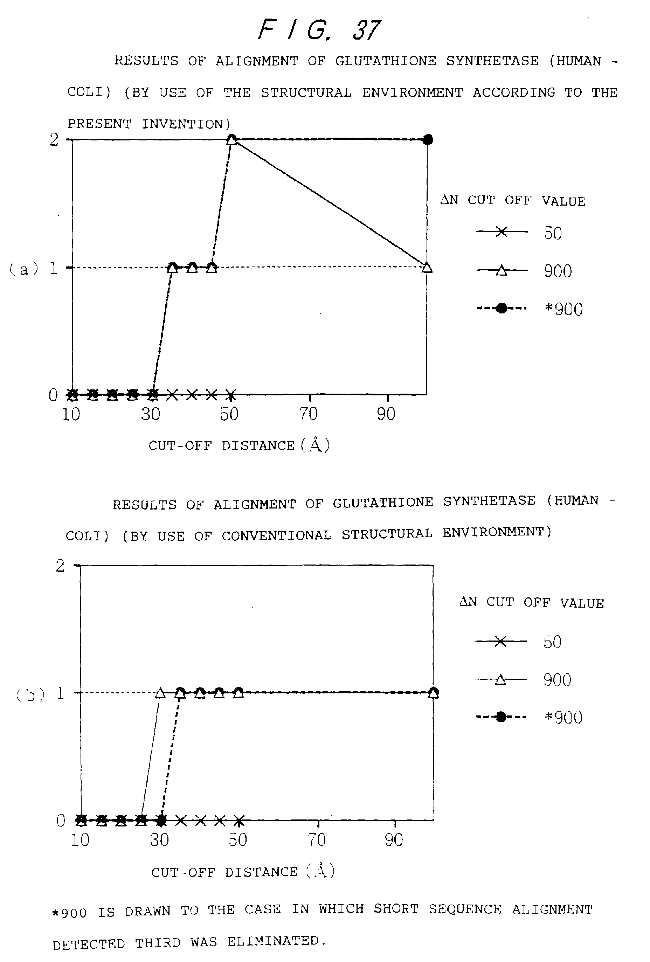

FIG. 37 shows the results of structural local alignment of human glutathione synthetase and Escherichia coli glutathione synthetase under various cut-off distances and ΔN cut-off values. FIG. 37(a) shows the alignment results obtained by using structural environments reconstructed according to the present invention. FIG. 37(b) shows the alignment results obtained by using conventional structural environments.

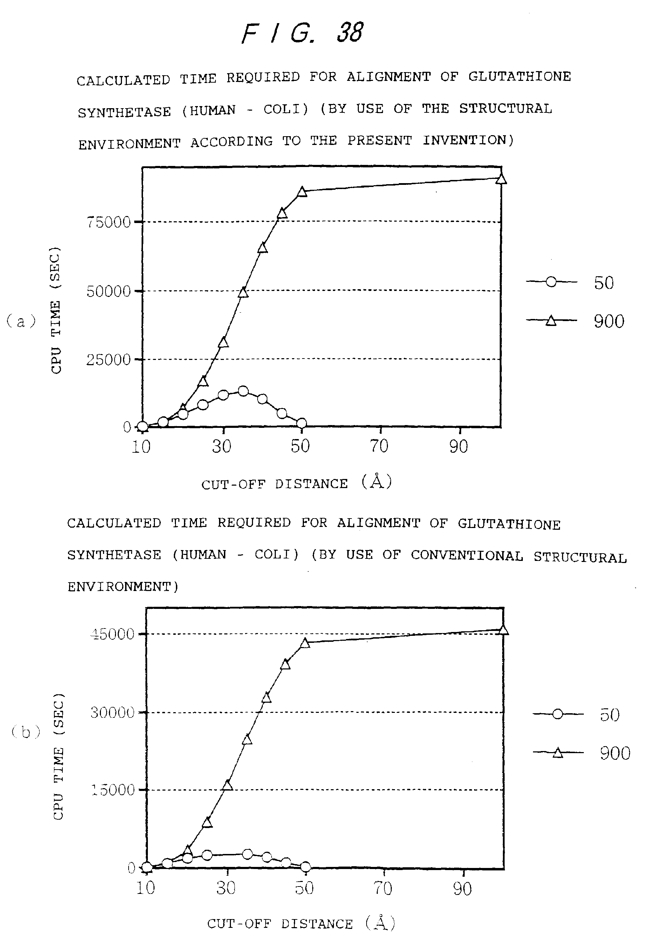

FIG. 38 shows the CPU time required to obtain the structural local alignments of human glutathione synthetase and Escherichia coli glutathione synthetase under various cut-off distances and ΔN cut-off values. FIG. 38(a) shows the computing time required to obtain the structural local alignments using the structural environments reconstructed according to the present invention. FIG. 38(b) shows the computing time required to obtain the structural local alignments using conventional structural environments.

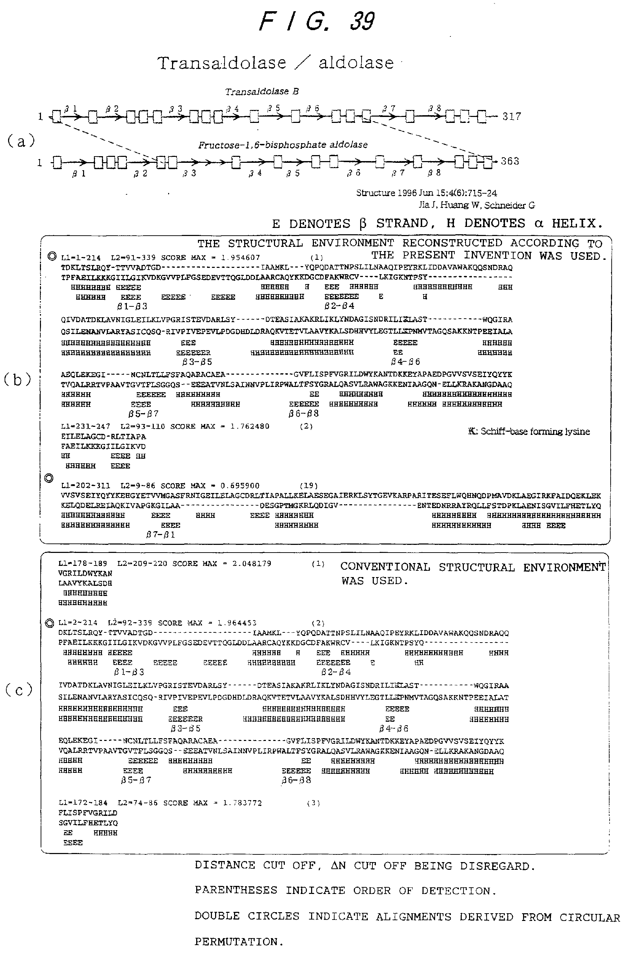

FIG. 39 shows the results of structural local alignment of transaldolase and fructose-1,6-bisphosphatealdolase with no consideration given to cut-off distance or ΔN cut-off. Among the structural local alignments obtained, alignments related to circular permutation are shown. FIG. 39(a) shows a schematic figure of transaldolase and fructose-1,6-bisphosphatealdolase. FIG. 39(b) shows the alignment results obtained by using structural environments reconstructed according to the present invention. FIG. 39(c) shows the alignment results obtained by using conventional structural environments.

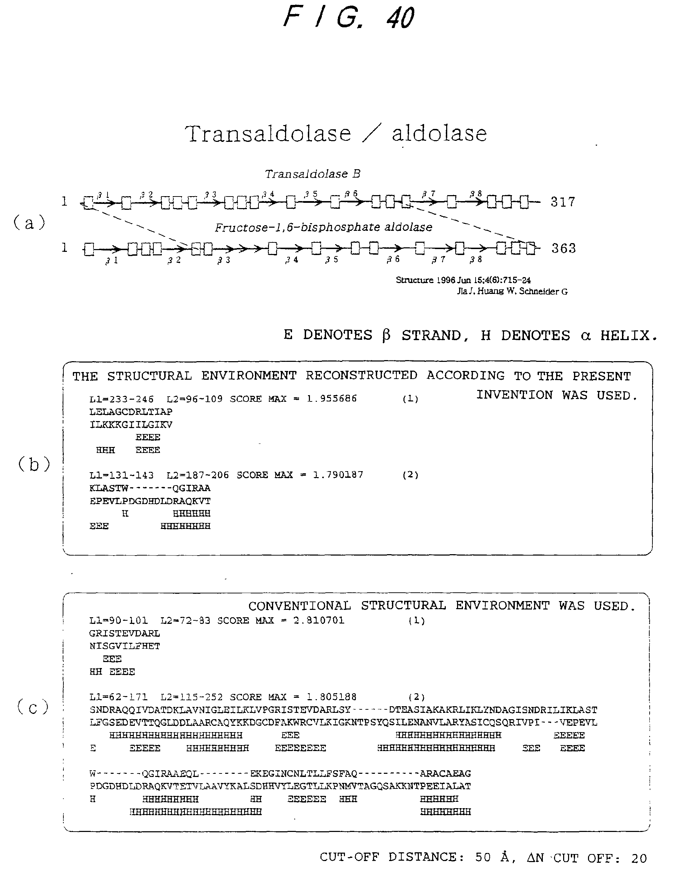

FIG. 40 shows the results of structural local alignment of transaldolase and fructose-1,6-bisphosphatealdolase under a cut-off distance of 20 Å and a ΔN cut-off of 20. FIG. 40(a) shows a schematic figure of transaldolase and fructose-1,6-bisphosphatealdolase. FIG. 40(b) shows the alignment results obtained by using structural environments reconstructed according to the present invention. FIG. 40(c) shows the alignment results obtained by using conventional structural environments.

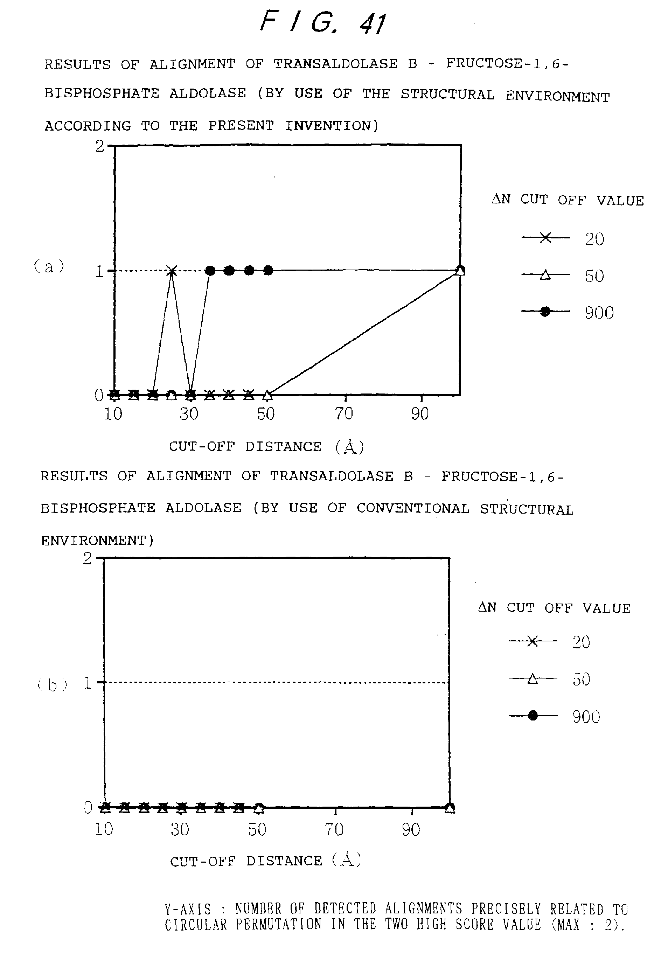

FIG. 41 shows the results of structural local alignment of transaldolase and fructose-1,6-bisphosphatealdolase under various cut-off distances and ΔN cut-off values. The vertical axis shows the detected alignment number related to circular permutation. FIG. 41(a) shows the alignment results obtained by using structural environments reconstructed according to the present invention. FIG. 41(b) shows the alignment results obtained by using conventional structural environments.

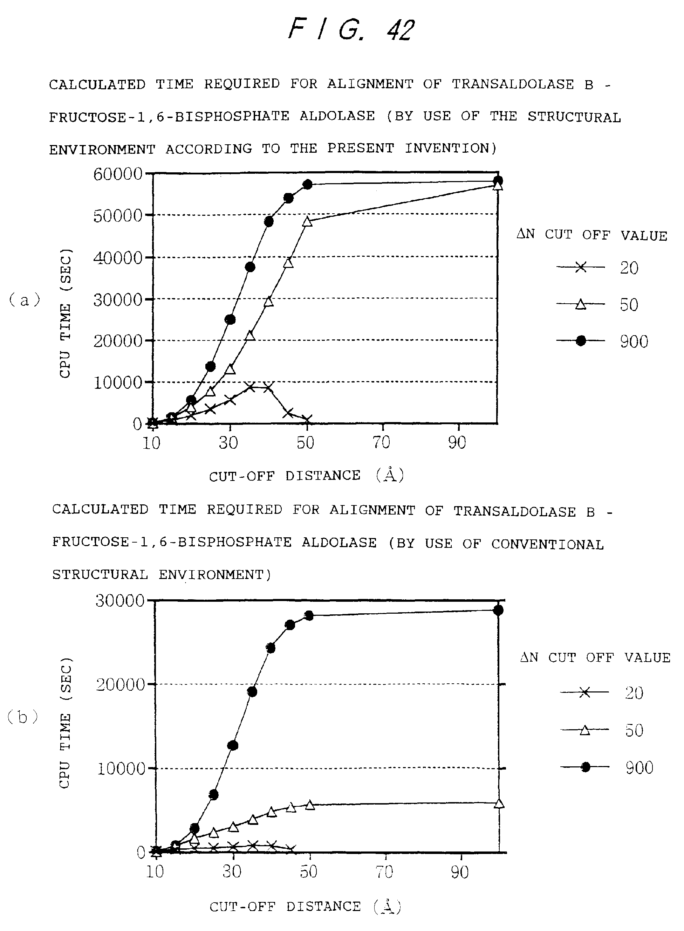

FIG. 42 shows the CPU time required to obtain the structural local alignments of transaldolase and fructose-1,6-bisphosphatealdolase under various cut-off distances and ΔN cut-off values. FIG. 42(a) shows the alignment computing time using the structural environments reconstructed according to the present invention. FIG. 42(b) shows the alignment computing time using conventional structural environments.

When the tertiary structures of proteins related to circular permutation are almost identical, upon structural local alignment using the environments of either the present invention or the conventional method, the obtained alignments with the maximum score and the second highest score were the circular permuted sequence.

However, concerning their score values, the structural environments according to the present invention showed for glucanase the top two scores of 12.14 and 10.89, and the third highest score of 1.65, and for streptavidin the top two scores of 5.13 and 4.40, and the third highest score of 1.49. Thus, in terms of score value, the method of the present invention clearly distinguished the very similar structures related to circular permutation from the other structures. In contrast, use of conventional environments showed that for glucanase, the top two scores were 12.05 and 7.12, and the third highest was 0.75; and for streptavidin the top two scores were 3.97 and 1.87, and the third highest score was 1.13. Thus, when conventional environments were used, the difference between the top two score values for structures related to circular permutation and the other structures was small as compared with those differences obtained by the present invention.

As is clearly shown by the figures of the molecules of FIGs. 23-24 and FIGs. 27-28, the tertiary structures of the two sequences related to circular permutation are very similar to each other. Therefore, it was expected that the top two alignments would show equally high score values upon structural local alignment; and the results obtained by reconstructed structural environments confirmed these expectations. Thus, it can be concluded that the structural environments reconstructed using the present invention have produced accurate alignment results.

Further, as indicated by alignments with cut-off distance and ΔN cut-off, when the structural environments obtained by the present invention were used, as shown by the glucanase example (see FIG. 24), alignment scores related to circular permutation showed the maximum and second highest values (10.6 and 9.27, respectively) and were clearly distinguishable from the third highest score value 1.03.

Also, in the case of streptavidin (see FIG. 28), the alignment scores related to circular permutation showed the highest and second highest score values of 4.38 and 3.54, respectively, which were also clearly distinguishable from the third highest score value of 1.78. In contrast, the conventional structural environments were completely unable to generate structural local alignments related to circular permutation.

FIGs. 25, 26, 29, and 30 show summaries of structural local alignment results and their computing times at various cut-off value settings with using the conventional structural environments or those related to the present invention. In the case of the examples shown in FIGs. 25 and 26 and FIGs. 29 and 30, even though their overall structures were almost identical, it was difficult to introduce a ΔN cut-off when conventional structural environments are used, thus clearly indicating that the similarities between the structural environments were not correctly expressed.

In contrast, when the structural environments obtained by the present invention were used, the similarities of overall structural environments were accurately presented, and even if ΔN cut-off was introduced, acceptably accurate structural local alignments were obtained. Concerning the computing time, as is the case with the proteins having locally similar structures to one another, the setting of cut-off distance at 10-20 Å is the condition under which the computation is carried out without loss in alignment accuracy as well as in a short period of time.

FIGs. 31-34, FIGs. 35-38, and FIGs. 39-42 show the results of structural local alignment for that group of proteins which are believed to be related to circular permutation (for FIGs. 31-34, Nat. Struct. Biol. (1998) Feb.: 5 (2): 101; for FIGs. 35-38, The EMBO Journal (1999) 18 (12): 3204-3213; and for FIGs. 39-42, Structure 1996 June 15: 4 (6): 715-724) though their structures differ from each other considerably.

In the case of the example of phthalate dioxygenase reductase and FMN-binding protein shown in FIGs. 31-34, the sizes of these proteins were largely different from each other; and thus, at a glance, they looked rather structurally different. However, by using reconstructed structural environments, both alignments related to circular permutation were detected. It is indicated that, even though their overall structures were different, only tertiary structures similar to each other were selected, and further that circular permutation was successfully detected.

In contrast, using conventional structural environments, even when structural local alignment was carried out disregarding cut-off distance and ΔN cut-off, only one alignment was detected but the other was not. When their structures were almost identical as the example of proteins that were artificially synthesized (FIGs. 23-26, and FIGs. 27-30), circular permutation was detected when structural local alignment was carried out using conventional structural environments without cut-off settings. However, when they were dissimilar (FIGs. 31-34), the structural environments reconstructed by the present invention are the only means by which the circular permutations can be detected.

From FIG. 33, it is also evident that, in case the molecular sizes of aligned proteins are considerably different to each other and similar regions in circular permutation are restricted to only small portions, the introduction of ΔN cut-off is not appropriate for the reason pointed out in the section on structural local alignments for ordinal proteins.

FIGs. 35 and 36 show an example of glutathione synthetase. Recently, the fact that human glutathione synthetase and bacterial glutathione synthetase have a circular permutation relationship to each other entered the literature. When the cut-off distance and ΔN cut-off conditions were not used, alignment results obtained by using reconstructed structural environments relating to the present invention could be used to detect one of the alignments generated by circular permutation, but were unable to detect the other, which was detected at the third highest score. However, at a 50 Å cut-off distance setting, the order of the alignment sequence unrelated to the circular permutation was replaced by the other alignment generated by circular permutation, and thus both of the circular permuted alignments were also detected. The above second highest scored alignment obtained without using cut-off was a short sequence consisting of slightly over 10 residues, which was detected at relatively high score values using environments generated by either the conventional method or the present invention, at various cut-off values. Since the sequence had no relation to circular permutation, the sequence was detected due probably to the presence of some similar structure. It is possible that a score at a very small local similar structure was overestimated because the scoring method employed by the present invention was obtained by dividing its alignment score by the sequence length so as not to become dependent on the sequence length. It should be pointed out that by using conventional structural environments the other alignment in circular permutation was not detected at all.

Further, as is the case with aldolase and transaldolase shown in FIGs. 39-42, in the case of circular permutation where even corresponding secondary structures were considerably different, upon structural local alignment by using structural environments of the present invention, only one alignment related to circular permutation could be detected accurately at the maximum score. In contrast, using conventional environments, the alignments involved in circular permutation were not detected at the maximum score.

From the above results, the following can be concluded: Compared with conventional structural environments, by carrying out local alignment using the reconstructed structural environments according to the present invention, accurate structural local alignments can be obtained even for the proteins having a relationship of circular permutation; to shorten computing time, a 10-35 Å cut-off distance setting is effective; and when the proteins to be compared are largely different in sequence length, then the ΔN cut-off setting is not appropriate.

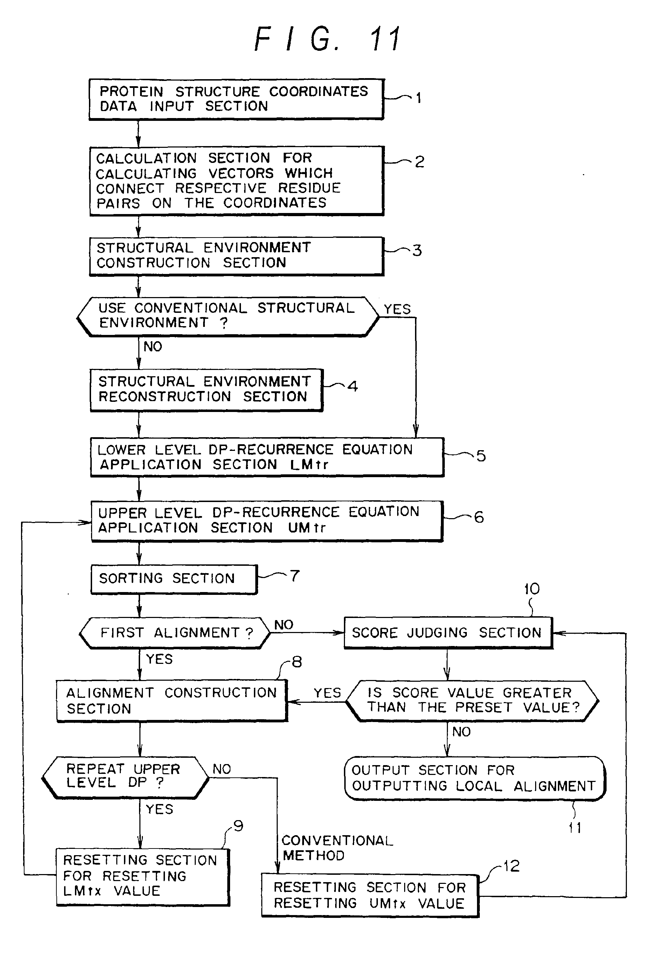

According to the present invention, using the method described above, even though there is a relationship of circular permutation between two proteins, their accurate structural local alignments can be obtained. This method will be elucidated with reference to FIG. 11, and the performance hardware used for this method will also be described.

FIG. 11 depicts a flow chart of the hardware employed to obtain structural local alignments using the double dynamic programming algorithm of the present invention. In FIG. 11, the following blocked sections are shown by numerals: 1, data input section for tertiary structure coordinates; 2, computing section for obtaining vectors connecting each residue pair of coordinate data; 3, construction section for structural environments; 4, reconstruction section for structural environments used to identify circular permutation; 5, application section of the lower level DP recurrence equation used in structural environment comparison; 6, application section of the upper level DP recurrence equation used for residue alignment; 7, sorting section for arranging each score obtained by the recurrence equation in order of score value from the highest; 8, alignment construction section for constructing structural local alignments by backtracking from the residue pair having the maximum score; 9, LMtx value resetting section where LMtr values at residues surrounding the residue pairs already used for alignments are reset to atypical values to avoid their repeated use for the next upper level DP; 10, score assessment section to determine the end of an alignment operation; 11, output section of structural local alignment results; and 12, resetting section of Umtx values to carry out backtracking using the method of Taylor et al. instead of repeating the upper level DP.

Structural local alignment using the double dynamic programming algorithm relating to the present invention is carried out in the order shown by the arrows. The coordinate data of protein tertiary structures are input at the data input section 1; then, vectors for coordinate data between paired residues are obtained at the vector computing section 2 for vectors to a particular atom at paired residues of coordinate data; and then their structural environments are constructed at the structural environment construction section 3.

When the cut-off distance approximation is introduced (Toh, H., CABIOS 13, 387-396 (1997)), it is introduced at the structural environment construction section 3. In case circular permutation is considered in calculation, the reconstructing section 4 for structural environments carries out reconstruction of those structural environments.

At the implementation section 5 of the lower level DP, first to avoid alignments between different secondary structures, residue pairs belonging to different secondary structures are omitted without lower level DP computation; and then their structural environment similarities are set to zero.

In the case of other residue pairs, if necessary, using the ΔN cut-off setting condition (Toh, H., CABIOS 13, 387-396 (1997)), the structural environments of the residues of two proteins are compared by calculating the lower level DP recurrence equation.

Next, the upper level DP recurrence equation is employed at the implementation section 6 of the upper level DP recurrence equation. Here, if necessary, to avoid gaps from being assigned among the sequences with secondary structure in aligning, operations as changing the gap penalty value on a secondary structure sequence can be added.

Subsequently, at the sorting section 7, the scores obtained by the recurrence equation from 6 above are realigned in order of score value from the highest, and then, at the alignment construction section 8 for constructing structural local alignments by backtracking, alignment construction is carried out by backtracking from the residue pair having the maximum score. Upon performing backtracking, at the LMtx value resetting section 9, among LMtr values, those at residue pairs within a certain width range along the backtracked path are reset to a negative value which would usually not occur.

Upon performing upper level DP again, omitting computation at the marked negative values results in disappearance of the path extended from the alignments already backtracked, thus leading to the discovery of another new structural local alignment together with a reduction in the computing time.

Next, by using the reset LMtx, the program returns to the application section 6 for the upper level DP recurrence equation, and starts re-computation by applying the upper level DP recurrence equation. By repeating these steps, more structural local alignments can be obtained.

At the score assessment section 10, the program judges the end of computation when the maximum score value in upper level DP after the second upper level DP steps becomes smaller than the set value. Then, at the final step, the obtained results are output by the output section 11 of structural local alignment results.

In FIG. 11, section 12 uses the same backtracking method adopted by Taylor and Orengo for the double dynamic programming algorithm, that is, by section 12, Umtr values at residues within a certain width range from residue pairs along the already backtracked path are reset to atypical negative values, and then, using the reset Umtr, the program returns to alignment construction section 8 by backtracking. Thus, by repeating the backtracking process, more structural local alignments can be obtained.

It should be noted that the present invention is not limited to the above examples. Based on the aim of the present invention, various modifications can be added, and thus these modifications are not to be excluded from the claims for the present invention.

As described in detail above, the present invention provides a structural local alignment method enabling carrying out accurate structural local alignments even between those proteins in circular permutation.

## Claims

1. A structural local alignment method for proteins using a double dynamic programming algorithm for maintaining constant presentation of the structural environments even though an exchange between the N-terminal and C-terminal regions takes place, which method comprises:

   (a) upon evaluation of the similarity in structure of two proteins, determining a structural environment as a vector directed from a certain atom (C$\alpha$ or C$\beta$) in each amino acid residue to a certain atom (C$\alpha$ or C$\beta$) in all the other amino acid residues;
   (b) in the above step (a), upon determination of a structural environment at amino acid residue i, establishing N-terminal and C-terminal vector sets, the N-terminal vectors being apposed in order of amino acid residue No. from a vector for an N-terminal residue to that for amino acid residue i - 1, and the C-terminal vectors being apposed in order of amino acid residue No. from a vector for amino acid residue i + 1 to that for the C-terminal;
   (c) reconstructing the structural environment so that the C-terminal vector set comes first and the N-terminal vector set comes next;
   (d) obtaining a first structural local alignment with the double dynamic programming algorithm using the structural environment constructed in the above step (c);
   (e) resetting, after obtaining the first structural local alignment, the similarity value of structural environment ($S_{i,j}$) to an anomalous value for each residue within a 5-10 residue width before and after a residue pair on the alignment path;
   (f) determining the second structural local alignment by backtracking after re-computing the upper level DP using the structural environment similarity values reset at the above step (e) while omitting pairing residues with $S_{i,j}$ that has been reset to the anomalous value; and
   (g) obtaining further structural local alignments by repeating the operation of the above steps (e) and (f) until the maximum score falls below the set value.

2. A structural local alignment method according to claim 1, which employs a distance cut-off operation for reducing computing time, the operation taking into consideration the protein local structure in the form of a structural environment; and secondary structure information of protein for improving alignment accuracy.

# *F I G. 1*

TWO-DIMENSIONAL MATRIX FOR CARRYING OUT DYNAMIC
PROGRAMMING ALGORITHM

AMINO ACID SEQUENCE OF PROTEIN B

AGHTLW -------------------- LEGNG
123456 --------- j------------------

AMINO ACID SEQUENCE OF PROTEIN A

ILTHGG
654321

$D_{i,j}$

LDGTG

ALGORITHM OF DYNAMIC PROGRAMMING (DP) IN
LOCAL ALIGNMENT

$$E_{i,j} = Max(D_{i,j-1} - a, E_{i,j-1} - b)$$
$$F_{i,j} = Max(D_{i-1,j} - a, F_{i-1,j} - b)$$
$$D_{i,j} = Max(S_{i,j} + D_{i-1,j-1}, E_{i,j}, F_{i,j}, O)$$

<EQUATION 1>

$$GAP\ PENALTY = a + b^{*}(K-1)$$

$S_{i,j}$ : SIMILARITY IN PHYSICOCHEMICAL PROPERTIES OF
AMINO ACID RESIDUE $i, j$

$a$ : OPEN GAP PENALTY, $b$ : EXTENTION GAP PENALTY
K  LENGTH OF GAP

$S_{i,j}$ ◄──────── SEE SCORE TABLE

# F I G. 2

$$E_{i,j} = \text{Max} (D_{i,j-1} - a, \quad E_{i,j-1} - b)$$

$$F_{i,j} = \text{Max} (D_{i-1,j} - a, \quad F_{i-1,j} - b)$$

$$D_{i,j} = \text{Max} (S_{i,j} + D_{i-1,j-1}, \quad E_{i,j}, \quad F_{i,j}, \quad 0 )$$

$S_{i,j}$ : similarity in physicochemical properties of amino acid residue $i$, $j$
a : open gap penalty
b : extention gap penalty
k : length of gap

WHEN $D_{i-1,j-1} + S_{i,j}$ IS CHOSEN AS MAX, A PATH FROM $D_{i-1,j-1}$ TO $D_{i,j}$ IS SELECTED. AMINO ACID RESIDUE i, j ; i.e., K AND R ARE ALIGNED.

WHEN $D_{i-1,j} - a$ OF $F_{i,j}$ IS CHOSEN AS MAX, IT IS SELECTED A PATH FROM $D_{i-1,j}$ TO $D_{i,j}$. AMINO ACID RESIDUE i AND A GAP ; i.e., R AND THE GAP ARE ALIGNED.

WHEN $F_{i-1,j} - b$ IS CHOSEN AS MAX, $D_{i,j}$ INDICATES THAT A GAP CONTINUES FROM A RESIDUE PAIR TO WHICH $F_{i-1,j}$ DIRECTS.

# F I G. 3

DAYM780301

D Log odds matrix for 250 PAMs (Dayhoff et al., 1978)
RA Dayhoff, M.O., Schwartz, R.M. and Orcutt, B.C.
TA model of evolutionary change in proteins
J In "Atlas of Protein Sequence and Structure", Vol.5, Suppl.3 (Dayhoff, M.O., ed.),
National Biomedical Research Foundation, Washington, D.C., p.352 (1978)

| | A | R | N | D | C | Q | E | G | H | I | L | K | M | F | P | S | T | W | Y | V |
|---|---|---|---|---|---|---|---|---|---|---|---|---|---|---|---|---|---|---|---|---|
| A | 2.0 | | | | | | | | | | | | | | | | | | | |
| R | -2.0 | 6.0 | | | | | | | | | | | | | | | | | | |
| N | 0.0 | 0.0 | 2.0 | | | | | | | | | | | | | | | | | |
| D | 0.0 | -1.0 | 2.0 | 4.0 | | | | | | | | | | | | | | | | |
| C | -2.0 | -4.0 | -4.0 | -5.0 | 12.0 | | | | | | | | | | | | | | | |
| Q | 0.0 | 1.0 | 1.0 | 2.0 | -5.0 | 4.0 | | | | | | | | | | | | | | |
| E | 0.0 | -1.0 | 1.0 | 3.0 | -5.0 | 2.0 | 4.0 | | | | | | | | | | | | | |
| G | 1.0 | -3.0 | 0.0 | 1.0 | -3.0 | -1.0 | 0.0 | 5.0 | | | | | | | | | | | | |
| H | -1.0 | 2.0 | 2.0 | 1.0 | -3.0 | 3.0 | 1.0 | -2.0 | 6.0 | | | | | | | | | | | |
| I | -1.0 | -2.0 | -2.0 | -2.0 | -2.0 | -2.0 | -2.0 | -3.0 | -2.0 | 5.0 | | | | | | | | | | |
| L | -2.0 | -3.0 | -3.0 | -4.0 | -6.0 | -2.0 | -3.0 | -4.0 | -2.0 | 2.0 | 6.0 | | | | | | | | | |
| K | -1.0 | 3.0 | 1.0 | 0.0 | -5.0 | 1.0 | 0.0 | -2.0 | 0.0 | -2.0 | -3.0 | 5.0 | | | | | | | | |
| M | -1.0 | 0.0 | -2.0 | -3.0 | -5.0 | -1.0 | -2.0 | -3.0 | -2.0 | 2.0 | 4.0 | 0.0 | 6.0 | | | | | | | |
| F | -4.0 | -4.0 | -4.0 | -6.0 | -4.0 | -5.0 | -5.0 | -5.0 | -2.0 | 1.0 | 2.0 | -5.0 | 0.0 | 9.0 | | | | | | |
| P | 1.0 | 0.0 | -1.0 | -1.0 | -3.0 | 0.0 | -1.0 | -1.0 | 0.0 | -2.0 | -3.0 | -1.0 | -2.0 | -5.0 | 6.0 | | | | | |
| S | 1.0 | 0.0 | 1.0 | 0.0 | 0.0 | -1.0 | 0.0 | 1.0 | -1.0 | -1.0 | -3.0 | 0.0 | -2.0 | -3.0 | 1.0 | 2.0 | | | | |
| T | 1.0 | -1.0 | 0.0 | 0.0 | -2.0 | -1.0 | 0.0 | 0.0 | -1.0 | 0.0 | -2.0 | 0.0 | -1.0 | -3.0 | 0.0 | 1.0 | 3.0 | | | |
| W | -6.0 | 2.0 | -4.0 | -7.0 | -8.0 | -5.0 | -7.0 | -7.0 | -3.0 | -5.0 | -2.0 | -3.0 | -4.0 | 0.0 | -6.0 | -2.0 | -5.0 | 17.0 | | |
| Y | -3.0 | -4.0 | -2.0 | -4.0 | 0.0 | -4.0 | -4.0 | -5.0 | 0.0 | -1.0 | -1.0 | -4.0 | -2.0 | 7.0 | -5.0 | -3.0 | -3.0 | 0.0 | 10.0 | |
| Z | 0.0 | -2.0 | -2.0 | -2.0 | -2.0 | -2.0 | -2.0 | -1.0 | -2.0 | 4.0 | 2.0 | -2.0 | 2.0 | -1.0 | -1.0 | -1.0 | 0.0 | -6.0 | -2.0 | 4.0 |

EP 1 098 257 A2

F I G. 4

## F I G. 5

N-TERMINAL  C-TERMINAL  N-TERMINAL  C-TERMINAL

PROTEIN A          PROTEIN B

STRUCTURAL
ENVIRONMENT

USING DP, SIMILARITY BETWEEN ELEMENTS IN TWO STRUCTURAL ENVIRONMENTS IS OBTAINED AND EXPRESSED AS $S_{i,j}$.

THE SIMILARITY BETWEEN TWO SEQUENCES CAN BE DETERMINED AS THE SCORE OF SEQUENCE GLOBAL ALIGNMENT.
THIS IDEA IS APPLIED TO THE SIMILARITY AMONG VECTORS THAT CONSTITUTE THE STRUCTURAL ENVIRONMENT.

(TAYLER & ORENGO)

PROTEIN B

PROTEIN A

$S_{i,j}$

LMtx

DP

SAME AS FIG. 3

PROTEIN B

PROTEIN A

$D_{i,j}$

UMtx

18

# F I G. 6

PROTEIN B

PROTEIN A

NEXT GREATEST SCORE

IF NOT RESET TO ZERO, ANY OF THESE VALUES BECOME GREATER THAN THAT OF THE NEXT LOCAL ALIGNMENT PATH.

WHEN ONE ALIGNMENT IS DETERMINED, THE VICINITY OF THE PATH IS SET TO ZERO.

THIS ELIMINATE GARBAGE PATHS, SHOWN IN THIN LINES NEAR THE ALIGNMENT.

# F I G. 7

GENE DUPLICATION

FUSING

DELETED                                          DELETED

DELETION

# F I G. 8

(1,3-1,4)-BETA-D-GLUCAN 4          CIRCULAR PERMUTED
     GLUCANOHYDROLASE          (EXPERIMENTALLY CREATED)

# F I G. 9

PROTEIN A

PROTEIN B

N-TERMINAL    C-TERMINAL

C-TERMINAL

N-TERMINAL

| STRUCTURAL ENVIRONMENT ON THE N-TERMINAL SIDE | i ⟶ 1 | i ⟶ 6 |
|---|---|---|
| | i ⟶ 2 | i ⟶ 7 |
| | i ⟶ 3 | i ⟶ 8 |
| | i ⟶ 4 | |
| | i ⟶ 5 | |
| | i ⟶ 6 | |
| | i ⟶ 7 | |
| | i ⟶ 8 | |

| STRUCTURAL ENVIRONMENT ON THE C-TERMINAL SIDE | i ⟶ 9 | i ⟶ 9 |
|---|---|---|
| | i ⟶ 10 | i ⟶ 10 |
| | | i ⟶ 1 |
| | | i ⟶ 2 |
| | | i ⟶ 3 |
| | | i ⟶ 4 |
| | | i ⟶ 5 |

## F I G. 10

# F I G. 11

PROTEIN STRUCTURE COORDINATES DATA INPUT SECTION — 1

CALCULATION SECTION FOR CALCULATING VECTORS WHICH CONNECT RESPECTIVE RESIDUE PAIRS ON THE COORDINATES — 2

STRUCTURAL ENVIRONMENT CONSTRUCTION SECTION — 3

USE CONVENTIONAL STRUCTURAL ENVIRONMENT ? — YES

NO

STRUCTURAL ENVIRONMENT RECONSTRUCTION SECTION — 4

LOWER LEVEL DP-RECURRENCE EQUATION APPLICATION SECTION LMtr — 5

UPPER LEVEL DP-RECURRENCE EQUATION APPLICATION SECTION UMtr — 6

SORTING SECTION — 7

FIRST ALIGNMENT ? — NO → SCORE JUDGING SECTION — 10

YES

ALIGNMENT CONSTRUCTION SECTION — 8

YES ← IS SCORE VALUE GREATER THAN THE PRESET VALUE?

NO

REPEAT UPPER LEVEL DP ? — NO

YES

OUTPUT SECTION FOR OUTPUTTING LOCAL ALIGNMENT — 11

CONVENTIONAL METHOD

RESETTING SECTION FOR RESETTING LMtx VALUE — 9

RESETTING SECTION FOR RESETTING UMtx VALUE — 12

23

EP 1 098 257 A2

# F I G. 12

| | PROTEIN DATA BANK |
|---|---|
| HIV-1 PROTEASE (E.C.3.4.23.-) | : 1HIH |
| ASPARTIC PROTEINASE (RHIZOPUSPEPSIN) | : 2APR |
| CYCLOOXYGENASE-2 (PROSTAGLANDIN SYNTHASE-2) | : 1CX2 |
| E-SELECTIN (LECTIN AND EGF DOMAINS, RESIDUES 1 - 157) | : 1ESL |
| IMMUNOGLOBULIN FAB   (LAMBDA LIGHT CHAIN) | : 7FAB |

# F I G. 13

(a)

HIV-1 PROTEASE        ASPARTIC PROTEINASE

HIV-1 protease

Acid proteinase

E DENOTES β STRAND, H DENOTES α HELIX.

(b)

```
                    THE STRUCTURAL ENVIRONMENT RECONSTRUCTED ACCORDING TO
◎ L1=9-145  L2=1-95 SCORE MAX = 2.623669      (1) THE PRESENT INVENTION WAS USED.
TDYGNDIEYYGQVTIGTPGKKFNLDFDTGSSDLWIASTLCTNCGSGQTKYDPNQSSTYQADGRTWSISYGDGSSASGILA
PQITLWQRPLVTIKIGG--QLKEALLDTGADDTVLEEMSL----------------PGRWKPKMIGGIGG-FIKVRQY
EEE    EEEEEEEE    EEEEEEEE    EEEE                      EEEEEEE  EEEEEEEE
       EEEEE       EEEEEEEE    EE                        EEEEEEEEE E EEEEEEE


K-DNVNLGGLLIKGQTIELAKREAASFASGPNDGLLGLGFDTITTVRGVKTEMDNLI
DQILIEICGHKAI-GTVLVGP---------TPVNIIGRN-----------LLTQIG
E EEEEE  EEE  EEEEEEEEEEHHHH    EEEE           HHHHHH
  EEEEE  EEEE EEEEE     .       EE HH         HH


◎ L1=184-313  L2=1-99 SCORE MAX = 2.503183      (2)
TTVPIDNSRGWWGITVDRATVGTSTVASSFDGILDTGTTLLILPNNIAASVARAYGASDNGDGTYTISCDTSAFKPLVFS
PQITLW---QRPLVT---IKIGGQ----LKEALLDTGADDTVLEEMSLP-----GRWKPKMIGGIGGFIKVRQYDQILIE
EEE       EEEEEEEEEE      EEEEEE    EEE HHHHHHHH    EEE        EEEE
          EE  EEE  E      EEEEEEEE      EE          EEEEEEEEE EEEEEEEE EEEE


INGASFQVSPDSLVFEEFQGQCIAGFGYGNWGFAIIGDTFLKNNYVVFN
ICGHKAIG---------------TVLVGPTPVNIIGRNLLTQIGCTLN
E  EEEEE HHHH  EEE  EEE         EEE HHH  EEEEEEE
E  EEEEE                EEEE    EE HHHH


L1=5-45  L2=53-87 SCORE MAX = 1.804757      (3)
```

(c)

```
                    CONVENTIONAL STRUCTURAL ENVIRONMENT WAS USED.
◎ L1=9-149  L2=1-96 SCORE MAX = 2.954435      (1)
TDYGNDIEYYGQVTIGTPGKKFNLDFDTGSSDLWIASTLCTNCGSGQTKYDPNQSSTYQADGRTWSISYGDGSSASGILA
PQITLWQRPLVTIKIGG--QLKEALLDTGADDTVLEEMSL-----------------PGRWKPKMIGGIGG-FIKVRQY
EEE    EEEEEEEE    EEEEEEEE    EEEE                       EEEEEEE  EEEEEEEE
       EEEEE       EEEEEEEE    EE                         EEEEEEEEE E EEEEEEE


K-DNVNLGGLLIKGQTIELAKREAASFASGPNDGLLGLGFDTITTVRGVKTEMDNLISQGL
DQILIEICGHKAI-GTVLVGP---------TPVNIIG--------------RNLLTQIGC
E EEEEE  EEE  EEEEEEEEEEHHHH    EEEE              HHHHHH
  EEEEE  EEEE EEEEE             EE                 HHHH


◎ L1=184-313  L2=1-99 SCORE MAX = 2.901170      (2)
TTVPIDNSRGWWGITVDRATVGTSTVASSFDGILDTGTTLLILPNNIAASVARAYGASDNGDGTYTISCDTSAFKPLVFS
PQITLW---QRPLVT---IKIGGQ----LKEALLDTGADDTVLEEMSLP-----GRWKPKMIGGIGGFIKVRQYDQILIE
EEE       EEEEEEEEEE      EEEEEE    EEE HHHHHHHH    EEE        EEEE
          EE  EEE  E      EEEEEEE       EE          EEEEEEEEE EEEEEEEE EEEE


INGASFQVSPDSLVFEEFQGQCIAGFGYGNWGFAIIGDTFLKNNYVVFN
ICGHKAIGT---------------VLVGPTPVNIIGRNLLTQIGCTLN
E  EEEEE HHHH  EEE  EEE     '   EEE HHH  EEEEEEE
E  EEEEEE                EEE    EE HHHH


L1=5-43  L2=19-67 SCORE MAX = 1.326784      (3)
```

THE UNDERLINED PORTIONS INDICATE THE SITES IN WHICH THE STRUCTURE IS
MOSTLY MAINTAINED.

DISREGARD OF DISTANCE CUT OFF, ΔN CUT OFF.
PARENTHESES INDICATE ORDER OF DETECTION.
DOUBLE CIRCLES INDICATE THE ALIGNMENTS OF
CORRESPONDING DOMAINS.

# F I G. 14

RESULTS OF ALIGNMENT OF HIV-1 PROTEASE-ASPARTIC PROTEINASE
(BY USE OF THE STRUCTURAL ENVIRONMENT ACCORDING TO THE
PRESENT INVENTION)

RESULTS OF ALIGNMENT OF HIV-1 PROTEASE-ASPARTIC PROTEINASE
(BY USE OF CONVENTIONAL STRUCTURAL ENVIRONMENT)

DETECTION OF TWO LOCAL
ALIGNMENTS IS EXPECTED.

Y-AXIS: NUMBER OF DETECTED
ALIGNMENTS WHICH HAVE BEEN
EXPECTED TO BE DETECTED IN
THE UPPER TWO PORTIONS.

# F I G. 15

CALCULATED TIME REQUIRED FOR ALIGNMENT OF HIV-1

PROTEASE-ASPARTIC PROTEINASE

(BY USE OF THE STRUCTURAL ENVIRONMENT ACCORDING TO THE PRESENT

INVENTION)

(a)

CALCULATED TIME REQUIRED FOR ALIGNMENT OF HIV-1

PROTEASE-ASPARTIC PROTEINASE

(BY USE OF CONVENTIONAL STRUCTURAL ENVIRONMENT)

(b)

# F I G. 16

(a)

EGF DOMAIN

Cyclooxygenase-2          E-Selectin (1-157: Lectin & EGF Domain )

EGF DOMAIN OF Cyclooxygenase-2 : ANPCCSNPCQNRGECMSTGFDQYKCDCTRTGFYGENCT
EGF DOMAIN OF E-Selectin       : YTAACTNTSCSGHGECVETINNYTCKCDPGFSGLKCE

(b)

```
                                    BY USE OF THE STRUCTURAL ENVIRONMENT
                                         ACCORDING TO THE PRESENT INVENTION
◎ L1=1-41  L2=119-157 SCORE MAX = 2.094741          (1)
  ANPCCSNPCQNRGECMSTGFDQYKCDCTRTGFYGENCTTP

  TAACTNTSCSGHGECVET-INNYTCKCDP-GFSGLKCEQI


  L1=531-552  L2=10-31 SCORE MAX = 1.658899          (2)
  ASIQSLICNNVKGCPFTSFNV
  MTYDEASAYCQQRYTHLVAIQ
```

(c)

```
                                 BY USE OF CONVENTIONAL STRUCTURAL ENVIRONMENT
◎ L1=2-41  L2=120-157 SCORE MAX = 2.269128          (1)
  NPCCSNPCQNRGECMSTGFDQYKCDCTRTGFYGENCTTP

  AACTNTSCSGHGECVET-INNYTCKCDP-GFSGLKCEQI


  L1=531-542  L2=13-24 SCORE MAX = 1.353986          (2)
  ASIQSLICNNV
  DEASAYCQQRY
```

BOLD PORTIONS ARE EGF MOTIF PORTIONS.
DOUBLE CIRCLES INDICATE ALIGNMENTS
CORRESPONDING TO EGF DOMAINS

DISTANCE CUT OFF
ΔN CUT OFF BEING DISREGARDED
PARENTHESES INDICATE ORDER OF DETECTION.

# F I G. 17

RESULTS OF ALIGNMENT OF CYCLOOXYGENASE-2 - E-SELECTIN (EGF DOMAIN)

(BY USE OF THE STRUCTURAL ENVIRONMENT ACCORDING TO THE PRESENT INVENTION)

(a)

ΔN CUT OFF VALUE

—✕— 20

—△— 50

—●— 900

CUT-OFF DISTANCE (Å)

RESULTS OF ALIGNMENT OF CYCLOOXYGENASE-2 - E-SELECTIN (EGF DOMAIN)

(BY USE OF THE CONVENTIONAL STRUCTURAL ENVIRONMENT)

(b)

ΔN CUT OFF VALUE

—✕— 20

—△— 50

—●— 900

CUT-OFF DISTANCE (Å)

DETECTION OF ONE LOCAL ALIGNMENT IS EXPECTED.

Y-AXIS : NUMBER OF DETECTED ALIGNMENTS WHICH HAVE BEEN EXPECTED TO BE DETECTED AT THE HIGHEST SCORE VALUE.

# F I G. 18

CALCULATED TIME REQUIRED FOR ALIGNMENT OF CYCLOOXYGENASE-2 -

E-SELECTIN (EGF DOMAIN)

(BY USE OF THE STRUCTURAL ENVIRONMENT ACCORDING TO THE

PRESENT INVENTION)

(a)

ΔN CUT OFF VALUE

—✕— 20

—△— 50

—●— 900

CALCULATED TIME REQUIRED FOR ALIGNMENT OF CYCLOOXYGENASE-2 -

E-SELECTIN (EGF DOMAIN)

(BY USE OF THE CONVENTIONAL STRUCTURAL ENVIRONMENT)

(b)

ΔN CUT OFF VALUE

—✕— 20

—△— 50

—●— 900

30

# F I G. 19

(a)

IMMUNOGLOBULIN FAB (LAMBDA LIGHT CHAIN)

(b)

```
                                                    BY USE OF THE STRUCTURAL ENVIRONMENT
L1-208-411  L2-4-204 SCORE MAX = 0.984671           ACCORDING TO THE PRESENT INVENTION
LEQSGPGLVRPSQTLSLTCTVS-GTSFDDYYWTWVRQPPGRGLEWIGYVFYTGTTLLDPSLRGRVTMLVNTSKMQFSLRLSSVTAADTAVYYCARNLIAGGIDVWGQ
LTQPPSVSGAPGQRVTISCTGSSSNIGAGHNVKWYQQLPGTAPKLLIFH------------NNARFSVSKSGTSATLAITGLQAEDEADYYCQSYD--RSLRVFGG
EEEE         EEEEEEEE       EEEEEEE       EEEEEEE   EEEEE   EEEEEE    EEEEEE         EEEEEEEE
EE     EEE   EEEEEE        EEEEE                    EEEEEEEEEEEEEEEEE          EEEEEEEEE      EEEE

GSLVTVSSASTKGPSVFPLAP--------TAALGCLVKDYPPEPVTVSWNSGA--LTSGVHTFPAVLQSSGLYSLSSVVTVPSSSLGTQ-TYICNVNHKPSNTKVDKK
GTKLTVLRQPKAAPSVTLFPPSSEELQANKATLVCLISDFYPGAVTVAWKADGSPVKAGVETTTPSKQSNNKYAASSYLSLTPEQWKSHKSYSCQVTHEGSTVEKTVA
EEEE         EEEEE        EEEEEEEEEE       EEEEE        EEE       EEE    EEEEEEEEE  HHHH   EEEEEEE      EEEEE
EEEE         EEEEE  HHHHH  EEEEEEEEEE       EEEEEE   EEE EEE       EEE    EEEEEEEEEE HHHH   EEEEEEE      EEEEEEE

L1-4-204  L2-208-411 SCORE MAX = 0.984671
LTQPPSVSGAPGQRVTISCTGSSSNIGAGHNVKWYQQLPGTAPKLLIFH------------NNARFSVSKSGTSATLAITGLQAEDEADYYCQSYD--RSLRVFGG
LEQSGPGLVRPSQTLSLTCTVS-GTSFDDYYWTWVRQPPGRGLEWIGYVFYTGTTLLDPSLRGRVTMLVNTSKMQFSLRLSSVTAADTAVYYCARNLIAGGIDVWGQ
EE     EEE   EEEEEE        EEEEE                    EEEEEEEEEEEEEEEEE          EEEEEEEEE      EEEE
EEEE         EEEEEEEE       EEEEEEE       EEEEEEE   EEEEE   EEEEEE    EEEEEE         EEEEEEEE

GTKLTVLRQPKAAPSVTLFPPSSEELQANKATLVCLISDFYPGAVTVAWKADGSPVKAGVETTTPSKQSNNKYAASSYLSLTPEQWKSHKSYSCQVTHEGSTVEKTVA
GSLVTVSSASTKGPSVFPLAP--------TAALGCLVKDYPPEPVTVSWNSGA--LTSGVHTFPAVLQSSGLYSLSSVVTVPSSSLGTQ-TYICNVNHKPSNTKVDKK
EEEE         EEEEE  HHHHH  EEEEEEEEEE       EEEEE        EEE   EEE       EEE    EEEEEEEEEE HHHH   EEEEEEE      EEEEEEE
EEEE         EEEEE        EEEEEEEEEE       EEEEE        EEE   EEE       EEE    EEEEEEEEEE HHHH   EEEEEEE      EEEEE

L1-65-147  L2-171-243 SCORE MAX = 0.216795
```

(c)

```
                   BY USE OF CONVENTIONAL STRUCTURAL ENVIRONMENT
L1-4-204  L2-208-411 SCORE MAX = 0.629073
LTQPPSVSGAPGQRVTISCTGSSSNIGAGHNVKWYQQLPGTAPKLLIFH------------NNARFSVSKSGTSATLAITGLQAEDEADYYCQSYD--RSLRVFGG
LEQSGPGLVRPSQTLSLTCTVSGTSFD-DYYWTWVRQPPGRGLEWIGYVFYTGTTLLDPSLRGRVTMLVNTSKMQFSLRLSSVTAADTAVYYCARNLIAGGIDVWGQ
EE     EEE   EEEEEE        EEEEE                    EEEEEEEEEEEEEEEEE          EEEEEEEEE      EEEE
EEEE         EEEEEEEE       EEEEEEE       EEEEEEE   EEEEE   EEEEEE    EEEEEE         EEEEEEEE

GTKLTVLRQPKAAPSVTLFPPSSEELQANKATLVCLISDFYPGAVTVAWKADGSPVKAGVETTTPSKQSNNKYAASSYLSLTPEQWKSHKSYSCQVTHEGSTVEKTVA
GSLVTVSSASTKGPSVFPLAP--------TAALGCLVKDYPPEPVTVSWNSGA--LTSGVHTFPAVLQSSGLYSLSSVVTVPSSSLGTQ-TYICNVNHKPSNTKVDKK
EEEE         EEEEE  HHHHH  EEEEEEEEEE       EEEEE   EEE EEE       EEE    EEEEEEEEEE HHHH   EEEEEEE      EEEEEEE
EEEE         EEEEE        EEEEEEEEEE       EEEEE        EEE   EEE    EEEEEEEEEE HHHH   EEEEEEE      EEEEE

L1-208-411  L2-4-204 SCORE MAX = 0.629073
LEQSGPGLVRPSQTLSLTCTVSGTSFD-DYYWTWVRQPPGRGLEWIGYVFYTGTTLLDPSLRGRVTMLVNTSKMQFSLRLSSVTAADTAVYYCARNLIAGGIDVWGQ
LTQPPSVSGAPGQRVTISCTGSSSNIGAGHNVKWYQQLPGTAPKLLIFH------------NNARFSVSKSGTSATLAITGLQAEDEADYYCQSYD--RSLRVFGG
EEEE         EEEEEEEE       EEEEEEE       EEEEEEE   EEEEE   EEEEEE    EEEEEE         EEEEEEEE
EE     EEE   EEEEEE        EEEEE                    EEEEEEEEEEEEEEEEE          EEEEEEEEE      EEEE

GSLVTVSSASTKGPSVFPLAP--------TAALGCLVKDYPPEPVTVSWNSGA--LTSGVHTFPAVLQSSGLYSLSSVVTVPSSSLGTQ-TYICNVNHKPSNTKVDKK
GTKLTVLRQPKAAPSVTLFPPSSEELQANKATLVCLISDFYPGAVTVAWKADGSPVKAGVETTTPSKQSNNKYAASSYLSLTPEQWKSHKSYSCQVTHEGSTVEKTVA
EEEE         EEEEE        EEEEEEEEEE       EEEEE        EEE   EEE    EEEEEEEEEE HHHH   EEEEEEE      EEEEE
EEEE         EEEEE  HHHHH  EEEEEEEEEE       EEEEEE   EEE EEE       EEE    EEEEEEEEEE HHHH   EEEEEEE      EEEEEEE

L1-19-345  L2-125-355 SCORE MAX = 0.103943
```

DISTANCE CUT OFF AND ΔN CUT OFF BEING DISREGARDED

PARENTHESES INDICATE ORDER OF DETECTION.
DOUBLE CIRCLES INDICATE THE ALIGNMENTS OF CORRESPONDING DOMAINS.

# F I G. 20

RESULTS OF ALIGNMENT OF IMMUNOGLOBULIN Fab DOMAINS

(BY USE OF THE STRUCTURAL ENVIRONMENT ACCORDING TO THE

PRESENT INVENTION)

(a)

ΔN CUT OFF VALUE

—△— 20

—●— 900

CUT-OFF DISTANCE (Å)

RESULTS OF ALIGNMENT OF IMMUNOGLOBULIN Fab DOMAINS

(BY USE OF CONVENTIONAL STRUCTURAL ENVIRONMENT)

(b)

ΔN CUT OFF VALUE

—△— 20

—●— 900

CUT-OFF DISTANCE (Å)

DETECTION OF TWO LOCAL ALIGNMENTS iS EXPECTED.
Y-AXIS : NUMBER OF DETECTED ALIGNMENTS WHICH HAVE BEEN
EXPECTED TO BE DETECTED AT THE TWO HIGH SCORES.

# F I G. 21

CALCULATED TIME REQUIRED FOR ALIGNMENT OF IMMUNOGLOBULIN Fab DOMAINS

(BY USE OF THE STRUCTURAL ENVIRONMENT ACCORDING TO THE

PRESENT INVENTION)

(a)

CALCULATED TIME REQUIRED FOR ALIGNMENT OF IMMUNOGLOBULIN Fab DOMAINS

(BY USE OF CONVENTIONAL STRUCTURAL ENVIRONMENT)

(b)

## F I G. 22

|  | PROTEIN DATA BANK |
|---|---|
| BACILLUS LICHENIFORMIS BETA-GLUCANASE | : 1GBG |
| CIRCULAR. PERMUTED (1-3,1-4)-BETA-D-GLUCAN | : 1AJK |
| | |
| AVIDIN COMPLEX WITH BIOTIN | : 1AVD |
| CIRCULAR PERMUTED STREPTAVIDIN E51/A46 | : 1SWG |
| | |
| PHTHALATE DIOXYGENASE REDUCTASE (E.C.1.18.1.) | : 2PIA |
| FMN-BINDING PROTEIN FROM DESULFOVIBRIO VULGARIS | : 1AXJ |
| | |
| HUMAN GLUTATHIONE SYNTHETASE | : 2HGS |
| ESCHERICHIA COLI GLUTATHIONE SYNTHETASE | : 1GSA |
| | |
| TRANSALDOLASE B | : 1ONR |
| FRUCTOSE-1,6-BISPHOSPHATE ALDOLASE (E.C.4.1.2.13) | : 1FBA |

EP 1 098 257 A2

# F I G. 23

(a)

1,3-1,4-beta-glucanase          circular permuted
                                1,3-1,4-beta-glucanase

1,3-1,4-beta-glucanase

circular permutated
1,3-1,4-beta-glucanase

Proteins 1998 Feb 1;30(2):155-67
Ay J, Hahn M, Heinemann U

E DENOTES β STRAND, H DENOTES α HELIX.

(b)

```
          THE STRUCTURAL ENVIRONMENT RECONSTRUCTED ACCORDING TO
◎ L1=86-214  L2=3-129 SCORE MAX = 12.137240      (1)THE PRESENT INVENTION
                                                    WAS USED.
GIVSSFFTYTGPTDGTPWDEIDIEFLGKDTTKVQFNYYTNGVGNHEKIVNLGFDAANSYHTYAFDWQPNSIKWYVDGQLK
GIVSSFFTYTGPAHGTQWDEIDIEFLGKDTTKVQFNYYTNGVGGHEKVISLGFDASKGFHTYAFDWQPGYIKWYVDGVLK
EEEEEEEEEE       EEEEEEEEE     EEEEEEE      EEE    HHHH EEEEEEE    EEEEEE
EEEEEEEEEE       EEEEEEEE      EEEEEEE      EEE         EEEEEEEE   EEEEEE


HTATTQIPQTPGKIMMNLWNGAGVDEWLGSYNGVTPLYAHYNWVRYTK
HTATANIPSTPGKIMMNLWNGTD--DWLGSYNGANPLYAEYDWVKYTS
EEE       EEEEEEEEE   HHHH         EEEEEEEEEEE
EEE       EEEEEEEEE              EEEEEEEEEEE


◎ L1=5-83  L2=134-212 SCORE MAX = 10.886431      (2)
SFYEPFNNYNTGLWQKADGYSNGNMFNCTWRANNVSMTSLGEMRLSLTSPSYNKFDCGENRSVQTYGYGLYEVNMKPA
SFFEPFNSYNSGTWEKADGYSNGGVFNCTWRANNVNFTNDGKLKLGLTSSAYNKFDCAEYRSTNIYGYGLYEVSMKPA
  EEE     EE  EEE            EE   EEEEE   EEEEEEE     EEEEEEE      EEEEEEE
  EEE     EE  EEE            EE   EEEEE   EEEEEEE     EEEEE    EEEEEEEEEEE


  L1=177-188  L2=2-13 SCORE MAX = 1.646572      (3)
```

(c)

```
          CONVENTIONAL STRUCTURAL ENVIRONMENT WAS USED.
◎ L1=86-214  L2=3-129 SCORE MAX = 12.054335    (1)
GIVSSFFTYTGPTDGTPWDEIDIEFLGKDTTKVQFNYYTNGVGNHEKIVNLGFDAANSYHTYAFDWQPNSIKWYVDGQLK
GIVSSFFTYTGPAHGTQWDEIDIEFLGKDTTKVQFNYYTNGVGGHEKVISLGFDASKGFHTYAFDWQPGYIKWYVDGVLK
EEEEEEEEEE       EEEEEEEEE     EEEEEEE      EEE    HHHH EEEEEEE   EEEEEE
EEEEEEEEEE       EEEEEEEE      EEEEEEE      EEE         EEEEEEEE  EEEEEE


HTATTQIPQTPGKIMMNLWNGAGVDEWLGSYNGVTPLYAHYNWVRYTK
HTATANIPSTPGKIMMNLWNGTD--DWLGSYNGANPLYAEYDWVKYTS
EEE       EEEEEEEEE   HHHH         EEEEEEEEEEE
EEE       EEEEEEEEE              EEEEEEEEEEE


◎ L1=5-83  L2=134-212 SCORE MAX = 7.124189      (2)
SFYEPFNNYNTGLWQKADGYSNGNMFNCTWRANNVSMTSLGEMRLSLTSPSYNKFDCGENRSVQTYGYGLYEVNMKPA
SFFEPFNSYNSGTWEKADGYSNGGVFNCTWRANNVNFTNDGKLKLGLTSSAYNKFDCAEYRSTNIYGYGLYEVSMKPA
  EEE     EE  EEE            EE   EEEEE   EEEEEEE     EEEEEEE     EEEEEEE
  EEE     EE  EEE            EE   EEEEE   EEEEEEE     EEEEE   EEEEEEEEEEE


  L1=143-170  L2=19-51 SCORE MAX = 0.754815      (3)
```

DISTANCE CUT OFF, ΔN CUT OFF BEING DISREGARD.

PARENTHESES INDICATE ORDER OF DETECTION.

DOUBLE CIRCLES INDICATE ALIGNMENTS DERIVED FROM CIRCULAR

PERMUTATION.

# F I G. 24

(a)

1,3-1,4-beta-glucanase  circular permuted
          1,3-1,4-beta-glucanase

```
                                                 84                    214
1,3-1,4-beta-glucanase        - - - - - - - -
circular permutated
1,3-1,4-beta-glucanase         1                    131
```

Proteins 1998 Feb 1;30(2):155-67

Ay J, Hahn M, Heinemann U

E DENOTES β STRAND, H DENOTES α HELIX.

(b)

```
       THE STRUCTURAL ENVIRONMENT RECONSTRUCTED ACCORDING TO
                                                        THE PRESENT INVENTION
◎ L1=85-214  L2=2-129  SCORE MAX = 10.612882        (1)WAS USED.
  VGIVSSFFTYTGPTDGTPWDEIDIEFLGKDTTKVQFNYYTNGVGNHEKIVNLGFDAANSYHTYAFDWQPNSIKWYVDGQL
  TGIVSSFFTYTGPAHGTQWDEIDIEFLGKDTTKVQFNYYTNGVGGHEKVISLGFDASKGFHTYAFDWQPGYIKWYVDGVL
  EEEEEEEEE          EEEEEEEEE      EEEEEE         EEE       HHHH EEEEEEE    EEEEEE
  EEEEEEEEEE  .      EEEEEEEE       EEEEEE         EEE            EEEEEEEE    EEEEEE

  KHTATTQIPQTPGKIMMNLWNGAGVDEWLGSYNGVTPLYAHYNWVRYTK
  KHTATANIPSTPGKIMMNLWNGTD--DWLGSYNGANPLYAEYDWVKYTS
  EEE        EEEEEEEEE    HHHH        EEEEEEEEEEE
  EEE        EEEEEEEEE                EEEEEEEEEEE

◎ L1=5-83   L2=134-212  SCORE MAX = 9.272789        (2)
  SFYEPFNNYNTGLWQKADGYSNGNMFNCTWRANNVSMTSLGEMRLSLTSPSYNKFDCGENRSVQTYGYGLYEVNMKPA
  SFFEPFNSYNSGTWEKADGYSNGGVFNCTWRANNVNFTNDGKLKLGLTSSAYNKFDCAEYRSTNIYGYGLYEVSMKPA
  EEE     EE  EEE         EE   EEEEE   EEEEEEE-  -EEEEEEE       EEEEEEEE
  EEE         EEE         EE   EEEEE   EEEEEEE     EEEEE        EEEEEEEEEEE

  L1=194-209  L2=54-69  SCORE MAX = 1.029149        (3)
```

(c)

```
            CONVENTIONAL STRUCTURAL ENVIRONMENT WAS USED.
  L1=100-212  L2=17-212  SCORE MAX = 4.379914        (1)
  GTPWDEIDIEFLGKDTTKVQFNYYTNGVGNHEKIVNLGFDAANSYHTYAFDWQPNSIKWYVDGQLKHTATTQIPQ-----
  GTQWDEIDIEFLGKDTTKVQFNYYTNGVGGHEKVISLGFDASKGFHTYAFDWQPGYIKWYVDGVLKHTATANIPSTPGKI
  EEEEEEEEE      EEEEEEE       EEE      HHHH SEEEEEE   EEEEEE      EEE
  EEEEEEEE       EEEEEEE       EEE           EEEEEEEE  EEEEEE      EEE         EEE

  -----------TPGKIMM------------------NLW--------------------N-----------------
  MMNLWNGTDDWLGSYNGANPLYAEYDWVKYTSNQTGGSFFEPFNSYNSGTWEKADGYSNGGVFNCTWRANNVNFTNDGKL
             EEEEE        EEEEEEEEEEE  .     EEE         EEE            EE   EEEEE   EE
  EEEEEE      EEEEE        EEEEEEEEEEE        EEE         EEE            EE   EEEEE   EE

  ---GAGVDEWLGS------YNGVTPLYAHYNWVRY
  KLGLTSSAYNKFDCAEYRSTNIYGYGLYEVSMKPA
             HHHH        EEEEEEEEEE
  EEEEE       EEEEE      EEEEEEEEEEE

◎ L1=5-16   L2=134-145  SCORE MAX = 1.420107        (2)
  SFYEPFNNYNT
  SFFEPFNSYNS
  EEE     EE
   EEE

  L1=203-214  L2=172-183  SCORE MAX = 1.147908       (3)
```

CUT-OFF DISTANCE: 20 Å, ΔN CUT OFF: 20

# F I G. 25

RESULTS OF ALIGNMENT OF 1,3-1,4-BETA-GLUCANASE-CIRCULAR

PERMUTED GLUCANASE

(BY USE OF THE STRUCTURAL ENVIRONMENT ACCORDING TO THE

PRESENT INVENTION)

(a)

ΔN CUT OFF VALUE

—✕— 10

—✚— 20

—△— 50

---●--- 900

CUT-OFF DISTANCE (Å)

RESULTS OF ALIGNMENT OF 1,3-1,4-BETA-GLUCANASE-CIRCULAR

PERMUTED GLUCANASE

(BY USE OF CONVENTIONAL STRUCTURAL ENVIRONMENT)

(b)

ΔN CUT OFF VALUE

—✕— 10

—✚— 20

—△— 50

---●--- 900

CUT-OFF DISTANCE (Å)

Y-AXIS : NUMBER OF DETECTED ALIGNMENTS PRECISELY RELATED TO
CIRCULAR PERMUTATION IN THE TWO HIGH SCORE VALUE (MAX : 2).

## F I G. 26

CALCULATED TIME REQUIRED FOR ALIGNMENT OF 1,3-1,4-BETA-

GLUCANASE-CIRCULAR PERMUTED GLUCANASE

(BY USE OF THE STRUCTURAL ENVIRONMENT ACCORDING TO THE

PRESENT INVENTION)

(a)

CALCULATED TIME REQUIRED FOR ALIGNMENT OF 1,3-1,4-BETA-

GLUCANASE-CIRCULAR PERMUTED GLUCANASE

(BY USE OF CONVENTIONAL STRUCTURAL ENVIRONMENT)

(b)

# F I G. 27

(a)

Avidin        circular   permuted
                     streptavidin

Avidin    1          42 43                    125

circular   permuted
streptavidin   1          79 80          132

Protein Sci 1998 Apr;7(4):848-59

Chu V, Stayton PS

E DENOTES β STRAND, H DENOTES α HELIX.

(b)

◎ L1=4~34  L2=83~112 SCORE MAX = 5.133983        (1)
LTGKWTNDLGSNMTIGAVNSRGEFTGTYTT
ITGTWYNQLGSTFIVT-AGADGALTGTYES
  EEEE    EEEEE     EEEEEEEE
  EEEEE    EEEEE EE  EEEEEEEE

THE STRUCTURAL ENVIRONMENT RECONSTRUCTED ACCORDING TO THE PRESENT INVENTION WAS USED.

◎ L1=43~121  L2=1~82 SCORE MAX = 4.403324        (2)
KESPLHGTENT--INKRTQPTFGFTVNWK----FSESTTVFTGQCFIDRNGKEVLKTMWLLRSSVNDIGDDWKATRVGINIFTR
SRYVLTGRYDSAPATDGSGTALGWTVAWKNNYRNAHSATTWSGQYVG--GAEARINTQWLLTSGT-TEANAWKSTLVGHDTFTK
  EEEEEE       EEEEE      EEEEEEEEEE    EEEEEEEEEEE     EEEEEEEEEE
  EEEEEE        EEEEEEEEE    EEEEEEEEEEE     EEEEEEEEE     EEEEEEEEEE

L1=46~69. L2=94~112 SCORE MAX = 1.486921        (3)

(c)

CONVENTIONAL STRUCTURAL ENVIRONMENT WAS USED.

◎ L1=43~121  L2=1~82 SCORE MAX = 3.968864        (1)
KESPLHGTENT--INKRTQPTFGFTVNWK----FSESTTVFTGQCFIDRNGKEVLKTMWLLRSSVNDIGDDWKATRVGINIFTR
SRYVLTGRYDSAPATDGSGTALGWTVAWKNNYRNAHSATTWSGQYVG--GAEARINTQWLLTSGT-TEANAWKSTLVGHDTFTK
  EEEEE       EEEEE      EEEEEEEEEE    EEEEEEEEEE     EEEEEEEEEE
  EEEEE       EEEEEEEEE    EEEEEEEEEEE     EEEEEEEEE     EEEEEEEEEE

◎ L1=4~34  L2=83~112 SCORE MAX = 1.865853        (2)
LTGKWTNDLGSNMTIGAVNSRGEFTGTYTT
ITGTWYNQLGSTFIVT-AGADGALTGTYES
  EEEE    EEEEE     EEEEEEEE
  EEEEE    EEEEE EE  EEEEEEEE

L1=3~98 L2=21~112 SCORE MAX = 1.130406        (3)

DISTANCE CUT OFF, ΔN CUT OFF BEING DISREGARD.

PARENTHESES INDICATE ORDER OF DETECTION.

DOUBLE CIRCLES INDICATE ALIGNMENTS DERIVED FROM CIRCULAR

PERMUTATION.

# F I G. 28

(a)

Avidin    circular permuted streptavidin

Avidin    1 •-••-••-••-••-••-• 42 43 •-••-••-••-••-••-•• 125

circular permuted streptavidin    1 •-••-••-••-••-• 79 80 •-••-••-• 132

Protein Sci 1998 Apr;7(4):848-59

Chu V, Stayton PS

E DENOTES β STRAND, H DENOTES α HELIX.

(b)

◎ L1=4-34  L2=83-112 SCORE MAX = 4.376377
LTGKWTNDLGSNMTIGAVNSRGEFTGTYTT
ITGTWYNQLGSTFIVT-AGADGALTGTYES
　　EEEE　　　EEEEE　　　EEEEEEEE
　　EEEEE　　EEEEEE EE　EEEEEEEE

THE STRUCTURAL ENVIRONMENT RECONSTRUCTED ACCORDING TO THE PRESENT INVENTION WAS USED.

◎ L1=43-121  L2=1-82 SCORE MAX = 3.538487
KESPLHGTENT--INKRTQPTFGFTVNWK----FSESTTVFTGQCFIDRNGKEVLKTMWLLRSSVNDIGDDWKATRVGINIFTR
SRYVLTGRYDSAPATDGSGTALGWTVAWKNNYRNAHSATTWSGQYVGGA--EARINTQWLLTSGT-TEANAWKSTLVGHDTFTK
　　EEEEE　　　　　　EEEEE　　　　　EEEEEEEEEE　　EEEEEEEEEEE　　　　EEEEEEEEEE
　　EEEEE　　　　　　EEEEEEEEE　　　EEEEEEEEEEE　　　EEEEEEEEE　　　　EEEEEEEEEE

L1=112-123  L2=1-12 SCORE MAX = 1.777277

(c)

Alignment=    CONVENTIONAL STRUCTURAL ENVIRONMENT WAS USED.
L1=98-123  L2=59-102 SCORE MAX = 1.727098
RSSVNDIGDDWKATRVG----------------INIFTRLR
LTSGTTEANAWKSTLVGHDTFTKGITGTWYNQLGSTFIVTAGA
　　　　　　EEEEE　　　　　　　　　EEEEE
EE　　　　EEEEEEEEEE　　EEEEE　　EEEEEEEE

L1=11-98  L2=2-112 SCORE MAX = 1.644737
DLGSNMTIGAVNS----RGEFTGTYTTAVTATSNEIKESPLHGTENTINKRTQPTFGFTVNWK----------FSESTT
RYVLTGRYDSAPATDGSGTALGWTVAWKNNYRNAH-SATTWSGQYVGGAE---ARINTQWLLTSGTTEANAWKSTLVGHD
　EEEEE　　　　　EEEEEEEE　　　　EEEEE　　　EEEEEE　　　　　EEE
　EEEEE　　　　　EEEEEEEEE　　E EEEEEEEEEE　　　EEEEEEEEE　　　EEEEEE

VF-----------TGQCFIDRNGKEVLKTMWLL
TFTKGITGTWYNQLGSTFIVTAGADGALTGTYES
EE　　　　EEEEE　　EEEEEEEEEE
EEE　　EEEEE　EEEEEEEE　EEEEEEEE

L1=6-64  L2=22-89 SCORE MAX = 1.442828

CUT-OFF DISTANCE: 20 Å, ΔN CUT OFF: 20

# F I G. 29

RESULTS OF ALIGNMENT OF AVIDIN - CIRCULAR PERMUTED
STREPTAVIDIN (BY USE OF THE STRUCTURAL ENVIRONMENT ACCORDING
TO THE PRESENT INVENTION)

(a)

CUT-OFF DISTANCE (Å)

ΔN CUT OFF VALUE
—✕— 10
—▷— 20
—✢— 50
—□— 75
—△— 100
—●— 900

RESULTS OF ALIGNMENT OF AVIDIN - CIRCULAR PERMUTED
STREPTAVIDIN (BY USE OF CONVENTIONAL STRUCTURAL ENVIRONMENT)

(b)

CUT-OFF DISTANCE (Å)

ΔN CUT OFF VALUE
—✕— 10
—▷— 20
—✢— 50
—□— 75
—△— 100
—●— 900

Y-AXIS : NUMBER OF DETECTED ALIGNMENTS PRECISELY RELATED TO
CIRCULAR PERMUTATION IN THE TWO HIGH SCORE VALUE (MAX : 2).

# F I G. 30

CALCULATED TIME REQUIRED FOR ALIGNMENT OF AVIDIN - CIRCULAR
PERMUTED STREPTAVIDIN (BY USE OF THE STRUCTURAL ENVIRONMENT
ACCORDING TO THE PRESENT INVENTION)

(a)

CALCULATED TIME REQUIRED FOR ALIGNMENT OF AVIDIN - CIRCULAR
PERMUTED STREPTAVIDIN

(BY USE OF CONVENTIONAL STRUCTURAL ENVIRONMENT)

(b)

## F I G. 31

(a)

Phthalate dioxygenase reductase          FMN binding protein

FAD-BINDING DOMAIN IS STRUCTURALLY SIMILAR TO FMN BINDING PROTEIN

Phthalate dioxygenase reductase

```
1              30                          120
```

FMN binding protein

```
1                   80       120
```

E DENOTES β STRAND, H DENOTES α HELIX.

(b)

```
                              THE STRUCTURAL ENVIRONMENT RECONSTRUCTED ACCORDING TO
◎ L1=40~115  L2=11~83  SCORE MAX = 2.630809      (1)      THE PRESENT INVENTION WAS USED.
EAGANLTVAVP---NGSRRTYSLC-NDSQERNRYVIAVKRDSNGRGGSISFIDDTSEGDAVEVSLPRNEFPLDKRAKSF
KNEGVVAIATQGEDGPHLVNTWNSYLKVLDGNRIVVPVG------GMHKTEANVARDERVLMTLGSRKVAGRNGPGTG
      EEEEEE      EEEEE      EEEEEEEEEE    HHHHHH    EEEEE            EE
      EEEEEEE      EEEEEEE      EEEEEE      HHHHHHHH EEEE            EE

◎ L1=6~28  L2=80~113  SCORE MAX = 2.115652      (2)
DGFLRLKIASKEKIAR-----------DIWSFE
GTGFLIR-GSAAFRTDGPEFEAIARFKWARAALV
      EEEEEEEEE  EE          EEEEE
   EEEEEE EEEEEE HHHHHHH      EEEE

   L1=7~63  L2=27~93  SCORE MAX = 1.986093      (3)
```

(c)

```
                              CONVENTIONAL STRUCTURAL ENVIRONMENT WAS USED.

◎ L1=32~113  L2=3~86  SCORE MAX = 2.114660      (1)
QGAPLPPFEAGANLTVAVP---NGSRRTYSLC-NDSQERNRYVIAVKRDSNGRGGSISFIDDTSEGDAVEVSLPRNEFPLDKRAKSFILV
PGTFFEVLKNEGVVAIATQGEDGPHLVNTWNSYLKVLDGNRIVVPVG------GMHKTEANVARDERVLMTLGSRKVAGRNGPGTGFLI
           EEEEE        EEEEE        EEEEEEEEEE    HHHHHH    EEEEE            EEEEE
   HHHHHH    EEEEEEE      EEEEEEE      EEEEEE      HHHHHHHH EEEE            EEEEE

   L1=6~85  L2=26~99  SCORE MAX = 1.584974      (2)
DGFLRLKIASKEKIARDIWSFELTDPQGAPLPPFEAGANLTVAVPNGSRRTYSLCNDSQERNRYVIAVKRDSNGRGGSI
PHLVNTWNSYLKVLDGNRIVVPVGGM-HKTEANVARDERVLMTLGSRKVAGRNGP-----GTGFLIRGSAAFRTDGPEF
      EEEEEEEEE  EEEEEEEEEE          EEEEE    EEEEE       EEEEEEEEE    HHH
   EEEEEE      EEEEE      HHHHHHHH EEEE                 EEEEEEEEEEE   HHHH

   L1=22~72  L2=62~114  SCORE MAX = 1.083777      (3)
```

DISTANCE CUT OFF, ΔN CUT OFF BEING DISREGARD.

# F I G. 32

(a)

Phthalate dioxygenase reductase          FMN binding protein

FAD-BINDING DOMAIN IS STRUCTURALLY SIMILAR TO FMN BINDING PROTEIN

Phthalate dioxygenase reductase

1                30                              120

1                          80        120

FMN binding protein

E DENOTES β STRAND, H DENOTES α HELIX.

(b)

THE STRUCTURAL ENVIRONMENT RECONSTRUCTED ACCORDING TO
THE PRESENT INVENTION WAS USED.

◎ L1=18-29  L2=103-114 SCORE MAX = 3.035336   (1)
KIARDIWSFEL
RFKWARAALVI
   EEEEEEEEE
    EEEEE

◎ L1=43-132  L2=14-100 SCORE MAX = 1.852402   (2)
ANLTVAVP---NGSRRTYSLC-NDSQERNRYVIAVKRDSNGRGGSISFIDDTSEGDAVEVSLPRNEFPLDKRAKSFILVAGGIGITPMLSMAR
GVVAIATQGEDGPHLVNTWNSYLKVLDGNRIVVPVG------GMHKTEANVARDERVLMTLGSRKVAGRNGPGTGFLIRGSAAFRTDGPEFE
EEEEEE      EEEEE         EEEEEEEEEE ·  HHHHHHH      EEEEE             EEEEEEE    HHHHHHHH
EEEEEEE      EEEEEEE        EEEEE         HHHHHHHHH EEEE             EEEEEEEEEEEE  HHHHH

L1=181-201  L2=51-71 SCORE MAX = 1.394626

(c)

CONVENTIONAL STRUCTURAL ENVIRONMENT WAS USED.

L1=181-199  L2=51-69 SCORE MAX = 1.657375   (1)
FDFWSVFEKSKPAQHVYC
MHKTEANVARDERVLMTL
   HHHHH       EEEE
 HHHHHHHHH EEEEEE

◎ L1=37-133  L2=8-101 SCORE MAX = 1.439757   (2)
PPFEAGANLTVAVP---NGSRRTYSLCNDSQERNRYVIAVKRDSNGRGGSISFIDDTSEGDAVEVSLPRNEFPLDKRAKSFILVAGGIG
EVLKNEGVVAIATQGEDGPHLVNTWNSYLKVLDG---NRIVVPVGGMHKTEANVARDE----RVLMTLGSRKVAGRNGPGTGFLIRGSAA
    EEEEEE      EEEEE        EEEEEEEEEE    HHHHHHH      EEEE           EEEEEE
    EEEEEEE      EEEEEEE         EEEEE   HHHHHHHH E      EEE           EEEEEEEEEE

ITPMLSMARQ
FRTDGPEFEA
HHHHHHHHHH
EE  HHHHH

L1=17-29  L2=81-93 SCORE MAX = 1.371275   (3)

CUT-OFF DISTANCE: 15 Å,

ΔN CUT OFF: 50

# F I G. 33

RESULTS OF ALIGNMENT OF PHTHALATE DIOXYGENASE REDUCTASE - FMN

BINDING PROTEIN (BY USE OF THE STRUCTURAL ENVIRONMENT

ACCORDING TO THE PRESENT INVENTION)

RESULTS OF ALIGNMENT OF PHTHALATE DIOXYGENASE REDUCTASE - FMN

BINDING PROTEIN (BY USE OF CONVENTIONAL STRUCTURAL ENVIRONMENT)

Y-AXIS : NUMBER OF DETECTED ALIGNMENTS PRECISELY RELATED TO
CIRCULAR PERMUTATION IN THE TWO HIGH SCORE VALUE (MAX : 2).

# F I G. 34

CALCULATED TIME REQUIRED FOR ALIGNMENT OF PHTHALATE
DIOXYGENASE REDUCTASE - FMN BINDING PROTEIN (BY USE OF THE
STRUCTURAL ENVIRONMENT ACCORDING TO THE PRESENT INVENTION)

(a)

CALCULATED TIME REQUIRED FOR ALIGNMENT OF PHTHALATE
DIOXYGENASE REDUCTASE - FMN BINDING PROTEIN (BY USE OF
CONVENTIONAL STRUCTURAL ENVIRONMENT)

(b)

# F I G. 35

(a)

(Human Glutathione synthetase)  (Escherichia Coli Glutathione synthetase)

E DENOTES β STRAND, H DENOTES α HELIX.

(b)

THE STRUCTURAL ENVIRONMENT RECONSTRUCTED ACCORDING TO THE PRESENT INVENTION WAS USED.

(c)

CONVENTIONAL STRUCTURAL ENVIRONMENT WAS USED.

DISTANCE CUT OFF, ΔN CUT OFF BEING DISREGARD.

PARENTHESES INDICATE ORDER OF DETECTION.

DOUBLE CIRCLES INDICATE ALIGNMENTS DERIVED FROM CIRCULAR PERMUTATION.

# F I G. 36

(a)

(Human Glutathione synthetase)  (Escherichia Coli Glutathione synthetase)

(b)

THE STRUCTURAL ENVIRONMENT RECONSTRUCTED ACCORDING TO THE PRESENT INVENTION WAS USED.

◎ L1=201~470  L2=2~249  SCORE MAX = 3.134301

(c)

CONVENTIONAL STRUCTURAL ENVIRONMENT WAS USED.

◎ L1=201~470  L2=2~249  SCORE MAX = 2.734032

CUT-OFF DISTANCE: 50 Å, ΔN CUT OFF WAS NOT CONSIDERED.

PARENTHESES INDICATE ORDER OF DETECTION.

DOUBLE CIRCLES INDICATE ALIGNMENTS DERIVED FROM CIRCULAR PERMUTATION.

# F I G. 37

RESULTS OF ALIGNMENT OF GLUTATHIONE SYNTHETASE (HUMAN - COLI) (BY USE OF THE STRUCTURAL ENVIRONMENT ACCORDING TO THE PRESENT INVENTION)

RESULTS OF ALIGNMENT OF GLUTATHIONE SYNTHETASE (HUMAN - COLI) (BY USE OF CONVENTIONAL STRUCTURAL ENVIRONMENT)

*900 IS DRAWN TO THE CASE IN WHICH SHORT SEQUENCE ALIGNMENT DETECTED THIRD WAS ELIMINATED.

## F I G. 38

CALCULATED TIME REQUIRED FOR ALIGNMENT OF GLUTATHIONE
SYNTHETASE (HUMAN - COLI) (BY USE OF THE STRUCTURAL
ENVIRONMENT ACCORDING TO THE PRESENT INVENTION)

(a)

CALCULATED TIME REQUIRED FOR ALIGNMENT OF GLUTATHIONE
SYNTHETASE (HUMAN - COLI) (BY USE OF CONVENTIONAL STRUCTURAL
ENVIRONMENT)

(b)

# F I G. 39

## Transaldolase / aldolase

*Transaldolase B*

(a)

*Fructose-1,6-bisphosphate aldolase*

Structure 1996 Jun 15;4(6):715-24
Jia J, Huang W, Schneider G

E DENOTES β STRAND, H DENOTES α HELIX.

(b)

THE STRUCTURAL ENVIRONMENT RECONSTRUCTED ACCORDING TO THE PRESENT INVENTION WAS USED.

```
◎ L1=1-214  L2=91~339 SCORE MAX = 1.954607      (1)
TDKLTSLRQY-TTVVADTGD------------------IAAMKL---YQPQDATTNPSLILNAAQIPEYRKLIDDAVAWAKQQSNDRAQ
TPPFAEILKKKGIILGIKVDGVVPLFGSEDEVTTQGLDDLAARCAQYKKDGCDFAKWRCV----LKIGKNTPSY----------------
  HHHHHHH HEEEE                        HHHHH   H  EEE HHHHH        HHHHHHHHHHHH        HHH
    HHHHH   EEEE    EEEEE     EEEEE  HHHHHHHHH    EEEEEEE    E     H
         β1-β3                                 β2-β4

QIVDATDKLAVNIGLEILKLVPGRISTEVDARLSY------DTEASIAKAKRLIKLYNDAGISNDRILIKLAST-----------WQGIRA
QSILENANVLARYASICQSQ-RIVPIVEPEVLPDGDHDLDRAQKVTETVLAAVYKALSDHHVYLEGTLLKPNMVTAGQSAKKNTPEEIALA
  HHHHHHHHHHHHHHHHHH     EEE       HHHHHHHHHHHHHHHH     EEEEE       HHHHH
  HHHHHHHHHHHHHHHHHH     EEEEEER    HHHHHHHHHHHHHHHHHH       EE         HHHHHHH
         β3-β5                                 β4-β6

AEQLEKEGI-----NCNLTLLFSFAQARACAEA--------------GVFLISPFVGRILDWYKANTDKKEYAPAEDPGVVSVSEIYQYYK
TVQALRRTVPAAVTGVTFLSGGQS--EEEATVNLSAINNVPLIRPWALTFSYGRALQASVLRAWAGKKENIAAGQN-ELLKRAKANGDAAQ
  HHHHHH      EEEEEE  HHHHHHHHH           EE   HHHHHHHH      HHHHHHHHHHHHHHHHHH
  HHHHH       EEEE      HHHHHHHHH        EEEEE  HHHHHHHHH      HHHHH HHHHHHHHHHHH
         β5-β7                        β6-β8

L1=231~247  L2=93-110 SCORE MAX = 1.762480      (2)
EILELAGCD-RLTIAPA                                          K: Schiff-base forming lysine
FAEILKKKGIILGIKVD
  HH      EEEE HH
  HHHHH    EEEE

◎ L1=202-311  L2=9-86 SCORE MAX = 0.695900       (19)
VVSVSEIYQYYKEHGYETVVMGASFRNIGEILELAGCDRLTIAPALLKELAESEGAIERKLSYTGEVKARPARITESEFLWQHNQDPMAVDKLAEGIRKFAIDQEKLEK
KELQDELREIAQKIVAPGKGILAA---------------DESGPTMGKRLQDIGV---------------ENTEDNRRAYRQLLFSTDPKLAENISGVILFHETLYQ
  HHHHHHHHHHHH     EEEE      HHHH    EEEE HHHHHHHH                      HHHHHHHH  HHHHHHHHHHHHHHHHHHHHH
  HHHHHHHHHHHHH      EEEE              HHHHHHHH                           HHHHHHHHHHH     HHHH EEEE
         β7-β1
```

(c)

```
L1=178-189  L2=209-220 SCORE MAX = 2.048179     (1)   CONVENTIONAL STRUCTURAL ENVIRONMENT
VGRILDWYKAN                                            WAS USED.
LAAVYKALSDH
  HHHHHHHH
  HHHHHHHH

◎ L1=2-214  L2=92-339 SCORE MAX = 1.964453        (2)
DKLTSLRQY-TTVVADTGD-------------------IAAMKL---YQPQDATTNPSLILNAAQIPEYRKLIDDAVAWAKQQSNDRAQQ
PFAEILKKKGIILGIKVDKGVVPLFGSEDEVTTQGLDDLAARCAQYKKDGCDFAKWRCV----LKIGKNTPSYQ-----------------
  HHHHHHH HEEEE                       HHHHHH  H  EEE HHHHHHHHH        HHHHHHHHHHHH        HHHH
    HHHHH   EEEE    EEEEE     EEEEE  HHHHHHHHH    EEEEEEE    E     HH
         β1-β3                                 β2-β4

IVDATDKLAVNIGLEILKLVPGRISTEVDARLSY------DTEASIAKAKRLIKLYNDAGISNDRILIKLAST-----------WQGIRAA
SILENANVLARYASICQSQ-RIVPIVEPEVLPDGDHDLDRAQKVTETVLAAVYKALSDHHVYLEGTLLKPNMVTAGQSAKKNTPEEIALAT
  HHHHHHHHHHHHHHHHH     EEE       HHHHHHHHHHHHHHHH      EEEEE       HHHHH
  HHHHHHHHHHHHHHHHHH    EEEEEER    HHHHHHHHHHHHHHHHHH       EE        HHHHHHH
         β3-β5                                 β4-β6

EQLEKEGI-----NCNLTLLFSFAQARACAEA-------------GVFLISPFVGRILDWYKANTDKKEYAPAEDPGVVSVSEIYQYYK
VQALRRTVPAAVTGVTFLSGGQS--EEEATVNLSAINNVPLIRPWALTFSYGRALQASVLRAWAGKKENIAAGQN-ELLKRAKANGDAAQ
  HHHH      EEEEEE  HHHHHHHHH           EE   HHHHHHHH      HHHHHHHHHHHHHHHHHH
  HHHHH      EEEE      HHHHHHHHH       EEEEE  HHHHHHHHH      HHHHH HHHHHHHHHHHH
         β5-β7                        β6-β8

L1=172-184  L2=74-86 SCORE MAX = 1.783772       (3)
FLISPFVGRILD
SGVILFHETLYQ
  EE   HHHHH
  EEEE
```

DISTANCE CUT OFF, ΔN CUT OFF BEING DISREGARD.

PARENTHESES INDICATE ORDER OF DETECTION.

DOUBLE CIRCLES INDICATE ALIGNMENTS DERIVED FROM CIRCULAR

PERMUTATION.

# F I G. 40

## Transaldolase / aldolase

*Transaldolase B*

(a)

1 ~ 317

*Fructose-1,6-bisphosphate aldolase*

1 ~ 363

Structure 1996 Jun 15;4(6):715-24
Jia J, Huang W, Schneider G

E DENOTES β STRAND, H DENOTES α HELIX.

(b)

```
THE STRUCTURAL ENVIRONMENT RECONSTRUCTED ACCORDING TO THE PRESENT
                                            INVENTION WAS USED.
  L1=233-246  L2=96-109 SCORE MAX = 1.955686      (1)
LELAGCDRLTIAP
ILKKKGIILGIKV
        EEEE
  HHH   EEEE


  L1=131-143  L2=187-206 SCORE MAX = 1.790187     (2)
KLASTW-------QGIRAA
EPEVLPDGDHDLDRAQKVT
       H        HHHHHH
EEE          HHHHHHHH
```

(c)

```
                       CONVENTIONAL STRUCTURAL ENVIRONMENT WAS USED.
  L1=90-101  L2=72-83 SCORE MAX = 2.810701          (1)
GRISTEVDARL
NISGVILFHET
    EEE
HH EEEE


  L1=62-171  L2=115-252 SCORE MAX = 1.805188        (2)
SNDRAQQIVDATDKLAVNIGLEILKLVPGRISTEVDARLSY------DTEASIAKAKRLIKLYNDAGISNDRILIKLAST
LFGSEDEVTTQGLDDLAARCAQYKKDGCDFAKWRCVLKIGKNTPSYQSILENANVLARYASICQSQRIVPI---VEPEVL
    HHHHHHHHHHHHHHHHHHHH        EEE        HHHHHHHHHHHHHHHHHHH     EEEEE
E     EEEEE     HHHHHHHHHH      EEEEEEEE        HHHHHHHHHHHHHHHHHHHHH    EEE    EEEE

W-------QGIRAAEQL--------EKEGINCNLTLLFSFAQ---------ARACAEAG
PDGDHDLDRAQKVTETVLAAVYKALSDHHVYLEGTLLKPNMVTAGQSAKKNTPEEIALAT
H         HHHHHHHHH        HH   EEEEEE  HHH       HHHHH
          HHHHHHHHHHHHHHHHHHHHH                    HHHHHHHH
```

CUT-OFF DISTANCE: 50 Å, ΔN CUT OFF: 20

EP 1 098 257 A2

# F I G. 41

RESULTS OF ALIGNMENT OF TRANSALDOLASE B - FRUCTOSE-1,6-
BISPHOSPHATE ALDOLASE (BY USE OF THE STRUCTURAL ENVIRONMENT
ACCORDING TO THE PRESENT INVENTION)

(a)

ΔN CUT OFF VALUE

—✕— 20

—△— 50

—●— 900

CUT-OFF DISTANCE (Å)

RESULTS OF ALIGNMENT OF TRANSALDOLASE B - FRUCTOSE-1,6-
BISPHOSPHATE ALDOLASE (BY USE OF CONVENTIONAL STRUCTURAL
ENVIRONMENT)

(b)

ΔN CUT OFF VALUE

—✕— 20

—△— 50

—●— 900

CUT-OFF DISTANCE (Å)

Y-AXIS : NUMBER OF DETECTED ALIGNMENTS PRECISELY RELATED TO
CIRCULAR PERMUTATION IN THE TWO HIGH SCORE VALUE (MAX : 2).

# F I G. 42

CALCULATED TIME REQUIRED FOR ALIGNMENT OF TRANSALDOLASE B - FRUCTOSE-1,6-BISPHOSPHATE ALDOLASE (BY USE OF THE STRUCTURAL ENVIRONMENT ACCORDING TO THE PRESENT INVENTION)

(a)

CALCULATED TIME REQUIRED FOR ALIGNMENT OF TRANSALDOLASE B - FRUCTOSE-1,6-BISPHOSPHATE ALDOLASE (BY USE OF CONVENTIONAL STRUCTURAL ENVIRONMENT)

(b)